# EUROPÄISCHE PATENTANMELDUNG

(11) **EP 4 670 649 A2**
(43) Veröffentlichungstag der Anmeldung: **31.12.2025**
(21) Anmeldenummer: 25214884.6
(22) Anmeldetag: 15.12.2020
(51) Int. Cl.: A61B 17/12

(54) **IMPLANTAT ZUR BEHANDLUNG VON ANEURYSMEN**

(30) Priorität: 23.12.2019 DE 102019135748; 10.07.2020 DE 102020118301
(62) Teilanmeldung aus: 20838923.9
(71) Anmelder: femtos GmbH, 44799 Bochum (DE)
(72) Erfinder: Monstadt, Hermann, 44797 Bochum (DE); Hannes, Ralf, 44137 Dortmund (DE); Henkes, Hans, 70192 Stuttgart (DE); Trösken, Volker, 58456 Witten (DE); Kontek, Ronald, 44805 Bochum (DE); Herklotz, Dennis, 42389 Wuppertal (DE); Germeroth, Dennis, 44623 Herne (DE)
(74) Vertreter: Schneiders & Behrendt Bochum

(57) **Zusammenfassung**

Die Erfindung betrifft Implantat (1) zur Behandlung arteriovenöser Fehlbildungen, insbesondere Aneurysmen (2), wobei das Implantat (1) in einem komprimierten Zustand durch einen Mikrokatheter (3) an einen Bestimmungsort im Blutgefäßsystem eines Patienten bringbar und dem Implantat (1) eine Sekundärstruktur aufgeprägt ist, durch die es bei Freisetzung aus dem Mikrokatheter (3) einen expandierten Zustand einnimmt, wobei das Implantat (1) über eine Ablösestelle (4) abtrennbar mit einer Einführhilfe (5) verbunden ist und im expandierten Zustand einen Grundkörper (6) aufweist, der aus Streben (16) aufgebaut ist, wobei die Streben (16) zumindest teilweise untereinander an Kreuzungspunkten verbunden sind, sodass sich zwischen den Streben (16) Zwischenräume (11) ergeben, wobei sich die Streben (16) im expandierten Zustand am proximalen Ende des Grundkörpers (6) nach radial außen und im weiteren Verlauf axial in distaler Richtung und radial nach innen erstrecken, sodass sich eine Ausbauchung des Grundkörpers (6) ergibt, wobei der Grundkörper (6) am distalen Ende eine Zone aufweist, an der die Streben (16) keine Verbindung zueinander aufweisen. Das erfindungsgemäße Implantat (1) ist in der Lage, sich gut an die Form des jeweiligen Aneurysmas (2) anzupassen.

## Beschreibung

Die Erfindung betrifft ein Implantat zur Behandlung arteriovenöser Fehlbildungen, insbesondere Aneurysmen, wobei das Implantat in einem komprimierten Zustand durch einen Mikrokatheter an einen Bestimmungsort im Blutgefäßsystem eines Patienten bringbar und dem Implantat eine Sekundärstruktur aufgeprägt ist, durch die es bei Freisetzung aus dem Mikrokatheter einen expandierten Zustand einnimmt, wobei das Implantat über eine Ablösestelle abtrennbar mit einer Einführhilfe verbunden ist.

Aneurysmen sind zumeist sackartig ausgebildete oder spindelförmige (fusiforme) Erweiterungen der Gefäßwand, die vornehmlich an strukturell geschwächten Stellen der Gefäßwand durch den konstanten Druck des Blutes entstehen. Entsprechend empfindlich und anfällig für Verletzungen sind die Gefäßinnenwände eines Aneurysmas. Die Ruptur eines Aneurysmas führt in der Regel zu erheblichen gesundheitlichen Beeinträchtigungen, im Falle zerebraler Aneurysmen zu neurologischen Ausfällen bis hin zum Tod des Patienten.

Neben chirurgischen Eingriffen, bei denen beispielsweise das Aneurysma mittels eines Clips abgeklemmt wird, sind insbesondere endovaskuläre Methoden zur Behandlung von Aneurysmen bekannt, wobei in erster Linie zwei Ansätze verfolgt werden. Zum einen kann das Aneurysma mit Okklusionsmitteln, insbesondere sog. Coils (Platinspiralen) ausgefüllt werden. Die Coils fördern die Thrombusbildung und sorgen somit für einen Verschluss des Aneurysmas. Zum anderen ist bekannt, den Zugang zum Aneurysma, etwa den Hals eines Beerenaneurysmas, von der Blutgefäßseite aus durch stentähnliche Implantate zu verschließen und vom Blutfluss abzukoppeln. Beide Verfahren dienen dazu, den Blutfluss in das Aneurysma und somit den Druck auf das Aneurysma zu vermindern, im Idealfall zu eliminieren und somit die Gefahr einer Ruptur des Aneurysmas zu reduzieren.

Bei der Verfüllung eines Aneurysmas mit Coils kann es vorkommen, dass die Verfüllung des Aneurysmas unzureichend ist, was die Blutzufuhr in das Aneurysma und somit einen weiterhin bestehenden Druck auf dessen Innenwand zulässt. Die Gefahr einer stetigen Erweiterung des Aneurysmas und schließlich seiner Ruptur besteht weiter, wenn auch in abgeschwächter Form. Darüber hinaus ist die Behandlungsmethode in erster Linie für Aneurysmen mit relativ engem Hals - sogenannten Beerenaneurysmen - geeignet, da ansonsten die Gefahr besteht, dass die Coils aus einem weiten Aneurysmahals in das Blutgefäß ragen und dort thrombogenisieren, was zu Verschlüssen im Gefäß führen kann. Schlimmstenfalls wird ein Coil vollständig aus dem Aneurysma geschwemmt und verschließt Gefäße an anderer Stelle. Um die Coils an ihrem Platz im Aneurysmasack zu halten, wird der Aneurysmahals häufig zusätzlich mit einem speziellen Stent abgedeckt.

Ein anderer intravaskulärer Behandlungsansatz setzt auf sogenannte Flussumlenker (Flow Diverter). Diese Implantate ähneln in ihrer äußeren Erscheinung Stents, die zur Behandlung von Stenosen eingesetzt werden. Da die Aufgabe der Flow Diverter jedoch nicht das Offenhalten eines Gefäßes, sondern der Verschluss des Aneurysmazugangs auf Seiten des Blutgefäßes ist, ist die Maschenweite sehr eng, alternativ sind diese Implantate mit einer Membran überzogen. Nachteilig bei diesen Implantaten ist die Gefahr, dass abgehende Seitenäste in unmittelbarer Nähe des zu behandelnden Aneurysmas mitunter ebenfalls abgedeckt und dadurch mittel- oder langfristig verschlossen werden.

In der WO 2012/034135 A1 ist ein Implantat offenbart, das sich aus einem ersten und einem zweiten Abschnitt zusammensetzt, welche innerhalb eines Katheters hintereinander angeordnet sind, nach Freisetzung innerhalb des Aneurysmas hingegen eine dreidimensionale, annähernd sphärische Form annimmt und so das Aneurysma ausfüllt. Ausgangsmaterial für das dreidimensionale Implantat ist ein maschenartiges Gewebe, die Ausführungsbeispiele und Abbildungen sind allesamt auf ein röhrenartiges Geflecht aus Formgedächtnismaterial bezogen. Als nachteilig hat sich bei diesem Stand der Technik herausgestellt, dass es eine unvorteilhafte Steifigkeit aufweist. Aneurysmen sind selten absolut rund, ein dreidimensionales Implantat sollte aber in der Lage sein, sich in die Morphologie des Aneurysmas bestmöglich einzupassen. Zudem ist das Implantat für Katheter geringen Kalibers zu voluminös.

Aus der WO 2017/089451 A1 ist ein weiteres Implantat zur Einbringung in Aneurysmen bekannt. Bei diesem setzt sich das Implantat aus mehreren Untereinheiten zusammen, die jeweils ein Gerüst aus Streben aufweisen, zwischen denen sich eine Bespannung befindet. Das Implantat muss innerhalb eines Aneurysmas allerdings zunächst mehrere Windungen ausbilden, bis eine ausreichende Abdeckung der Oberfläche des Aneurysmas erreicht ist.

Die WO 2009/135166 A2 offenbart ein Implantat für Aneurysmen, das fassartig und aus einer Vielzahl von miteinander verflochtenen Filamenten aufgebaut ist, die am proximalen und distalen Ende zentral in einem Punkt zusammenlaufen. Mit Hilfe eines solchen Implantats ist es möglich, ein Aneurysma mit einem einzelnen Implantat zu verschließen. Auf der anderen Seite kann der Blutstrom bewirken, dass die fassartige Struktur eingedrückt wird, sodass das Gesamtvolumen des Implantats abnimmt. Hiermit verbunden ist eine unvollständige Ausfüllung des Aneurysmas.

Allgemein stellt sich das Problem, dass auf der einen Seite ein Implantat wünschenswert wäre, welches alleine in der Lage ist, ein Aneurysma zu füllen. Auf der anderen Seite sind Aneurysmen jedoch häufig unregelmäßig geformt und weisen eine unterschiedliche Größe auf, weshalb ein einzelnes Implantat gut in der Lage sein müsste, sich an diese unterschiedlichen Formen anzupassen. Gerade wegen dieser Problematik werden häufig noch herkömmliche Coils verwendet, weil diese weitgehend selbständig den Raum des Aneurysmas ausfüllen und sich dabei verhaken, wobei der behandelnde Arzt in Abhängigkeit von der Größe des Aneurysmas die Zahl der eingebrachten Coils anpasst.

Ausgehend vom zuvor beschriebenen Stand der Technik stellt sich somit die Aufgabe, ein Implantat zur Verfügung zu stellen, das als einzelnes Implantat oder zusammen mit weiteren Okklusionsmitteln in der Lage ist, ein Aneurysma auszufüllen, sich dabei jedoch an die Form und Größe des Aneurysmainneren anpasst.

Diese Aufgabe wird erfindungsgemäß gemäß einer ersten Ausführungsform gelöst durch ein Implantat zur Behandlung arteriovenöser Fehlbildungen, insbesondere Aneurysmen, wobei das Implantat in einem komprimierten Zustand durch einen Mikrokatheter an einen Bestimmungsort im Blutgefäßsystem eines Patienten bringbar und dem Implantat eine Sekundärstruktur aufgeprägt ist, durch die es bei Freisetzung aus dem Mikrokatheter einen expandierten Zustand einnimmt, wobei das Implantat über eine Ablösestelle abtrennbar mit einer Einführhilfe verbunden ist und wobei das Implantat im expandierten Zustand einen Grundkörper aufweist, der über ein proximales und ein distales Segment verfügt, wobei das proximale und das distale Segment kuppelförmig ausgebildet sind, wobei die konvexe Seite der Kuppel des proximalen Segments in Richtung proximal und die konvexe Seite der Kuppel des distalen Segments in Richtung distal weist, und wobei das proximale und das distale Segment über mehrere Verbindungsstreben miteinander verbunden sind.

Dadurch, dass das Implantat über eine distale und eine proximale Kuppel verfügt, zwischen denen sich Verbindungsstreben erstrecken, weist das Implantat eine hohe Flexibilität auf. Diese ist sowohl in axialer als auch radialer Richtung gegeben, d. h. das Implantat kann sich in Längsrichtung an den vorhandenen Raum anpassen, aber auch in Rotationsrichtung um die Längsachse. Das Implantat kann im expandierten Zustand geringfügig größer sein als der Innenraum des Aneurysmas, die Flexibilität, die durch die in Längsrichtung zwischen distaler und proximaler Kuppel verlaufenden Verbindungsstreben herbeigeführt wird, erlaubt jedoch eine Anpassung des Implantats. Beispielsweise können die Verbindungsstreben leicht gestaucht oder verdreht werden, um eine Anpassung in axialer bzw. radialer Richtung zu erreichen. Durch die in der Regel leicht übermäßige Dimensionierung gegenüber dem zu behandelnden Aneurysma fixiert sich das Implantat darin selbst. Die Verbindungsstreben dienen in diesem Zusammenhang dem Längenausgleich und der Anpassung an das Aneurysma. Durch die Federwirkung erfolgt eine gute Verankerung.

Als Längsrichtung wird die Richtung entsprechend oder parallel zur von proximal nach distal verlaufenden Achse verstanden, wobei als in Längsrichtung verlaufende Verbindungsstreben auch solche verstanden werden, die zwar eine Längskomponente aufweisen, jedoch nicht geradlinig in Längsrichtung verlaufen. Es ist vielmehr vorteilhaft, wenn die Verbindungsstreben zwischen proximalem und distalem Segment kurvenlinienförmig verlaufen, weil auf diese Weise eine Flexibilität hinsichtlich der Länge erreicht wird. Die Verbindungsstreben können sich leicht strecken, indem sie eine geradlinigere Konfiguration annehmen, sie können jedoch auch gestaucht werden, wenn sich die Krümmung verstärkt. Die Verbindungsstreben erzeugen somit eine gewisse Federwirkung, sowohl in axialer als auch in radialer Richtung. Die Federwirkung verbessert die Verankerung des Implantats im Aneurysma. Als kurvenlinienförmiger Verlauf wird ein Verlauf der Verbindungsstreben mit einer oder mehreren Krümmungen verstanden.

Die einzelnen Verbindungsstreben verlaufen hinsichtlich der Längsrichtung im Wesentlichen parallel, d. h. die Verbindungsstreben weisen normalerweise keine Schnittpunkte auf und sind nicht miteinander verflochten, sodass sie unabhängig voneinander gestaucht oder gestreckt werden können. Ebenso ist eine Drehbewegung des distalen Segments gegenüber dem proximalen Segment um die Längsachse möglich, d. h. die Verbindungsstreben stellen auch eine radiale Flexibilität sicher. Ein im Wesentlichen paralleler Verlauf der Verbindungsstreben in Längsrichtung liegt erfindungsgemäß auch dann vor, wenn sich der Kurvenlinienverlauf der einzelnen Verbindungsstreben unterscheidet.

Die Begriffe "proximal" und "distal" sind so zu verstehen, dass beim Einbringen des Implantats zum behandelnden Arzt weisende Teile als proximal, vom behandelnden Arzt weg weisende Teile als distal bezeichnet werden. Das Implantat wird somit typischerweise durch einen Mikrokatheter in distaler Richtung vorgeschoben. Der Mikrokatheter kann z. B. ein Mikrokatheter mit einem Innendurchmesser von 0,021", 0,027" oder ggf. 0,033" sein. Der Ausdruck "axial" bezieht sich auf die Längsachse des Implantats, die bei gestreckt vorliegendem Implantat von proximal nach distal verläuft; "radial" bezeichnet eine hierzu orthogonale Richtung.

Die kuppelförmig ausgebildeten proximalen und distalen Enden des Grundkörpers können auch als tellerförmig bezeichnet werden. Typischerweise sind die im Endbereich vorliegenden Segmente leicht zu den jeweiligen Enden hin abgerundet, wodurch sie sich besser an die Form des Aneurysmas anpassen. Darüber hinaus ist eine abgerundete Form besonders atraumatisch. Die konvexe Wölbung der Kuppeln bzw. Teller weist somit zum distalen bzw. proximalen Ende des Grundkörpers, die konkave Wölbung hingegen in Richtung Grundkörperinneres. Ggf. können die proximalen und distalen Segmente auch abgeschrägt statt abgerundet sein, wobei in diesem Fall die typischerweise relativ flache Spitze als konvexe Seite verstanden wird. Insbesondere der proximale Teller muss jedoch nicht vollständig abgerundet sein; häufig ist zwar eine Abrundung vorhanden, die jedoch zentral in Richtung proximal einen Fortsatz aufweist, in dem der proximale Teller zur Ablösestelle hin ausläuft. Die Durchmesser von distalem und proximalem Segment im expandierten Zustand sind variabel und können sich an die Gegebenheiten anpassen, sodass unterschiedliche Aneurysmagrößen und Aneurysmahalsgrößen behandelt werden können. Das Implantat ist normalerweise so ausgelegt, dass es bei freier Expansion einen größeren Durchmesser annehmen würde als der Innenraum des Aneurysmas. Entsprechend fixiert sich das Implantat kraftschlüssig selbst im Aneurysma.

Zweckmäßigerweise sind das proximale und/oder das distale Segment aus miteinander zumindest teilweise verbundenen Rahmenstreben aufgebaut. Insbesondere können diese Rahmenstreben im proximalen und/oder distalen Segment eine Maschen- oder Schlaufenstruktur ausbilden. Die Rahmenstreben schneiden sich somit an bestimmten Kreuzungspunkten, sodass sich zwischen den Rahmenstreben Zwischenräume ergeben. Diese Zwischenräume bzw. Maschen/Schlaufen können unterschiedliche Formen aufweisen und beispielsweise waben-, rauten- oder blütenförmig sein, wobei die Kanten abgerundet sein können. Abgerundete Formen weisen den Vorteil auf, dass sie besonders atraumatisch sind. Die Zwischenräume müssen jedoch nicht komplett von Streben umgeben sein, vielmehr können die Schlaufen auch z. B. zu einer Seite eine Öffnung aufweisen, was die Flexibilität des Segments weiter erhöht. Beispielsweise kann das Implantat von distal bzw. proximal betrachtet 3 bis 8 kreisförmig angeordnete Schlaufen aufweisen.

Der Grundkörper des Implantats setzt sich somit hinsichtlich seiner Grundstruktur vorzugsweise im Wesentlichen aus Rahmenstreben, die das proximale und das distale Segment ausbilden, und Verbindungsstreben zusammen, welche das proximale und das distale Segment miteinander verbinden. Das Strebendesign gewährleistet die einfache Ein- und Entfaltung des Implantats zur Platzierung im Mikrokatheter bzw. zur Freisetzung aus demselben.

Die Verbindungsstreben verbinden das proximale und das distale Segment. Die Gesamtzahl der Verbindungsstreben kann z. B. 3 bis 10, insbesondere 6 bis 8 betragen. Die Verbindungsstellen zwischen Verbindungsstreben und distalem bzw. proximalem Segment, welche häufig aus Rahmenstreben aufgebaut sind, können in unterschiedlicher Weise angeordnet sein. Sie können beispielsweise in einer Ebene liegen, aber auch gegeneinander versetzt sein. Auch die Länge und die Form der Verbindungsstreben können gleich, aber auch unterschiedlich sein. So kann es bei versetzt angeordneten Verbindungsstellen notwendig sein, auch die Länge der Verbindungsstreben unterschiedlich zu wählen. Auch der Querschnitt der Verbindungsstreben kann je nach Einsatzzweck unterschiedlich gewählt werden.

Die Streben (Rahmenstreben und Verbindungsstreben) des Implantats können insbesondere durch Laserschneidtechniken erzeugt werden. Möglich ist jedoch auch der Aufbau des Grundkörpers als geflochtene Struktur, bei der einzelne Streben miteinander verflochten oder verwoben werden. Auch andere Herstellungsverfahren sind denkbar, beispielsweise eine galvanische oder lithographische Herstellung, 3D-Druck oder Rapid Prototyping. Die Streben können einen runden, ovalen, quadratischen oder rechteckigen Querschnitt aufweisen, wobei im Falle eines quadratischen oder rechteckigen Querschnitts die Kanten abgerundet sein können. Die einzelnen Streben können sich auch aus mehreren, miteinander verwundenen oder auch parallel verlaufenden Einzelfilamenten zusammensetzen.

Sinnvollerweise kann das distale Segment einen Bereich aufweisen, der frei ist von Rahmenstreben, wobei der Bereich expandierbar und komprimierbar ist. Vorzugsweise ist dieser Bereich im distalen Segment zentral angeordnet, befindet sich also ungefähr am distalen Ende des Grundkörpers. Im Falle der bevorzugten Maschen-/Schlaufenstruktur verfügt das distale Segment somit über eine Öffnung zwischen den Maschen/Schlaufen. Dieser Bereich erlaubt eine Anpassung der Größe des distalen Segments an die Erfordernisse im Aneurysma, insbesondere des Umfangs. In Abhängigkeit vom konkreten Einsatzzweck können unterschiedliche Größen und eine unterschiedliche Zahl der Maschen/Schlaufen gewählt werden. Je nach Größe und Form des Aneurysmas kann sich das distale Segment mehr oder weniger stark aufweiten oder komprimiert werden, wobei die Flexibilität über den (zentralen) Bereich ohne Rahmenstreben herbeigeführt wird. Der Bereich erzeugt somit eine gewisse Federwirkung.

Besonders bevorzugt ist es, das proximale Segment mit einer das proximale Segment zumindest teilweise abdeckenden Membran auszustatten. Auf diese Weise deckt die Membran den Aneurysmahals ab und verhindert weitgehend den Einstrom von Blut in das Aneurysma. Durch die Abkopplung des Aneurysmas vom Blutstrom verödet dieses schließlich und die Gefahr einer Ruptur des Aneurysmas ist gebannt. Die Membran hat somit eine blutstrommodulierende Wirkung.

Die Membran muss nicht auf das proximale Segment beschränkt sein, möglich ist auch das Versehen des distalen Segments mit einer Membran und/oder die Abdeckung der Verbindungsstreben mit einer Membran. Soweit sich im distalen Segment wie oben beschrieben ein Bereich befindet, der frei ist von Rahmenstreben, sollte dieser Bereich auch frei sein von der Membran. Durch weitere Membranen wird die blutstrommodulierende Wirkung der Membran(en) verstärkt. Darüber hinaus wird die Gefahr eines Endoleaks, d. h. des Einströmens von Blut durch ein Leck zwischen Implantat und Aneurysmasack, weiter reduziert.

Gemäß einer zweiten Ausführungsform betrifft die Erfindung ein Implantat zur Behandlung arteriovenöser Fehlbildungen, insbesondere Aneurysmen, wobei das Implantat in einem komprimierten Zustand durch einen Mikrokatheter an einen Bestimmungsort im Blutgefäßsystem eines Patienten bringbar und dem Implantat eine Sekundärstruktur aufgeprägt ist, durch die es bei Freisetzung aus dem Mikrokatheter einen expandierten Zustand einnimmt, wobei das Implantat über eine Ablösestelle abtrennbar mit einer Einführhilfe verbunden ist und im expandierten Zustand einen Grundkörper aufweist, der aus Streben aufgebaut ist, wobei die Streben zumindest teilweise untereinander an Kreuzungspunkten verbunden sind, sodass sich zwischen den Streben Zwischenräume ergeben, wobei sich die Streben im expandierten Zustand am proximalen Ende des Grundkörpers nach radial außen und im weiteren Verlauf axial in distaler Richtung und radial nach innen erstrecken, sodass sich eine Ausbauchung des Grundkörpers ergibt, wobei der Grundkörper am distalen Ende eine Zone aufweist, an der die Streben keine Verbindung zueinander aufweisen. Insbesondere kann der Grundkörper im expandierten Zustand am distalen Ende eine Öffnung aufweisen.

Gemäß dieser zweiten Ausführungsform hat der Grundkörper im expandierten Zustand die Gestalt einer geschlossenen Tulpenblüte. Die Streben laufen am proximalen Ende zentral an der Ablösestelle zusammen. Von der Ablösestelle ausgehend verlaufen die Streben zunächst in radialer Richtung nach außen und anschließend weiter in distaler Richtung, wobei sie sich zugleich der zentralen Längsachse wieder annähern. Unter Umständen können die Streben in dem Abschnitt, in dem sie in radialer Richtung nach außen verlaufen, auch einen Verlauf in proximaler Richtung aufweisen, wodurch sich für den Grundkörper insgesamt eine Einstülpung am proximalen Ende ergibt. Die Streben sind untereinander zumindest teilweise verbunden, sodass sich zwischen den Kanten der Streben Zwischenräume oder Maschen ausbilden, die unterschiedliche Formen aufweisen können, beispielsweise eine Waben-, Herz-, Blatt- oder Rautenform, wobei die Kanten der Zwischenräume/Maschen in der Regel abgerundet sind. Untereinander verbundene Streben können Segmente bilden, die den Blütenblättern einer Tulpe ähneln, d. h. vom proximalen Ende des Grundkörpers gehen mehrere blütenblattartige Segmente aus, die über den Umfang verteilt die äußere Oberfläche des Grundkörpers bilden und sich an die Innenwandung des Aneurysmas anlegen. Am proximalen Ende des Grundkörpers laufen die Segmente zusammen. Auf diese Weise wird ein Grundkörper geschaffen, bei dem die einzelnen Segmente elastisch und beweglich sind, sodass sie sich gut an die Form des Aneurysmas anpassen können, was eine atraumatische Implantation bewirkt. Die elastischen distalen Enden der Streben/Segmente dienen als Stütze, um das proximale Ende des Grundkörpers in den Aneurysmahals zu drücken, wodurch eine gute Abdichtung des Aneurysmas zum Blutstrom gefördert wird.

Am distalen Ende sind die Streben nicht miteinander verbunden, d. h. hier ist der Grundkörper offen. Allerdings können sich die Streben bzw. die von den Streben gebildeten Segmente am distalen Ende überlappen, sodass die Öffnung quasi geschlossen wird, die Öffnung am distalen Ende kann jedoch auch verbleiben, wenn die Streben/Segmente distal jeweils vor der Öffnung enden.

Insgesamt passt sich der Grundkörper weitgehend der Form des Aneurysmas an, d. h. die Streben erstrecken sich zunächst radial nach außen bis zur Aneurysmawandung und verlaufen anschließend an dieser entlang weiter nach distal mit einer Wölbung radial nach innen. Die genaue Form, die der Grundkörper im Aneurysma ausbildet, hängt somit auch von der Form des Aneurysmas ab. Ob am distalen Ende des Grundkörpers eine Öffnung verbleibt oder sich die Streben überlappen, hängt ebenfalls von der Form des Aneurysmas ab. Bei einem im Verhältnis zum Grundkörper eher kleinen Aneurysma können die Streben beispielsweise so weit zusammengedrückt werden, dass sich ihre distalen Enden überlappen und die Öffnung verschwindet, bei einem größeren Aneurysma kann hingegen eine distale Öffnung verbleiben. Das Implantat kann im expandierten Zustand geringfügig größer sein als der Innenraum des Aneurysmas, die Flexibilität, die durch die Streben herbeigeführt wird, erlaubt jedoch eine Anpassung des Implantats. Durch die in der Regel leicht übermäßige Dimensionierung gegenüber dem zu behandelnden Aneurysma fixiert sich das Implantat darin selbst. Die Form des Grundkörpers ähnelt der einer geschlossenen Tulpenblüte, kann jedoch auch als gestauchte Kugel- oder Ellipsoidform bezeichnet werden, wobei hierunter nicht lediglich die exakte geometrische Form verstanden wird, sondern auch eine Form, die der jeweiligen geometrischen Form zumindest nahekommt. Allgemein ist ein expandierter Grundkörper sinnvoll, der am proximalen Ende abgeflacht ist oder zentral eine Einstülpung nach innen aufweist, um zu verhindern, dass Teile des Implantats sich aus dem Aneurysma heraus in das Stammblutgefäß erstrecken.

Hinsichtlich der Streben für die zweite Ausführungsform der Erfindung gilt das zur ersten Ausführungsform gesagte, d. h. die Streben des Implantats können insbesondere durch Laserschneidtechniken erzeugt werden, möglich ist jedoch auch ein Grundkörper als geflochtene Struktur, bei der einzelne Streben miteinander verflochten oder verwoben werden. Auch andere Herstellungsverfahren sind denkbar, beispielsweise eine galvanische oder lithographische Herstellung, 3D-Druck oder Rapid Prototyping. Die Streben können einen runden, ovalen, quadratischen oder rechteckigen Querschnitt aufweisen, wobei im Falle eines quadratischen oder rechteckigen Querschnitts die Kanten abgerundet sein können. Die einzelnen Streben können sich auch aus mehreren, miteinander verwundenen oder auch parallel verlaufenden Einzelfilamenten zusammensetzen.

Auch gemäß der zweiten Ausführungsform können eine oder mehrere, den Grundkörper abdeckende Membranen vorgesehen werden. Besonders bevorzugt ist es, den proximalen Bereich des Grundkörpers mit einer zumindest teilweise abdeckenden Membran zu versehen. Auf diese Weise deckt die Membran den Aneurysmahals ab und verhindert weitgehend den Einstrom von Blut in das Aneurysma. Durch die Abkopplung des Aneurysmas vom Blutstrom verödet dieses schließlich und die Gefahr einer Ruptur des Aneurysmas ist gebannt. Die Membran hat somit eine blutstrommodulierende Wirkung.

Die Membran muss nicht auf den proximalen Bereich des Grundkörpers beschränkt sein, möglich ist auch das Versehen des distalen Bereichs mit einer Membran. Allerdings ist normalerweise das distale Ende, an dem sich bei nicht zu starker Überlappung der distalen Enden der Streben eine Öffnung befindet, frei von Membranen. Die blutstrommodulierende Wirkung der Membran(en) wird durch Vorsehen einer Membran in weiteren Bereichen des Grundkörpers verstärkt. Darüber hinaus wird die Gefahr eines Endoleaks, d. h. des Einströmens von Blut durch ein Leck zwischen Implantat und Aneurysmasack, weiter reduziert.

Unabhängig von der Ausführungsform ist das Implantat vorzugsweise zumindest teilweise aus Formgedächtnismaterialien gefertigt. Dies macht es möglich, dem Implantat die gewünschte Sekundärstruktur aufzuprägen, die es nach Verlassen des Mikrokatheters automatisch wieder einnimmt. Besonders Formgedächtnismetalle sind im Bereich der Medizintechnik hinlänglich bekannt; hervorzuheben sind diesbezüglich Nickel-Titan-Legierungen, wie sie unter dem Namen Nitinol verwendet werden. Die Einnahme der Sekundärstruktur bei Verlassen des Mikrokatheters erfolgt in der Regel infolge des Wegfalls des äußeren Zwangs, der durch den Mikrokatheter ausgeübt wird; möglich ist aber auch, dass sich die Sekundärstruktur aufgrund der Temperaturänderung bei Verlassen des Mikrokatheters ausbildet.

Das Implantat ist über eine Ablösestelle ablösbar mit einer Einführhilfe verbunden. Bei dieser Einführhilfe kann es sich um einen herkömmlichen Einführdraht handeln, mit dem das Implantat durch das Blutgefäßsystem an die gewünschte Stelle vorgeschoben werden kann. Besonders bevorzugt ist allerdings eine Einführhilfe, die rohr- oder schlauchförmig ausgebildet ist und ein inneres Lumen aufweist. Mit Hilfe einer solchen Einführhilfe lassen sich, nachdem der Grundkörper in das Aneurysma eingeführt und dort expandiert wurde, zusätzliche Okklusionsmittel in das Aneurysma bzw. das Innere des Grundkörpers einführen, die für einen zusätzlichen Verschluss des Aneurysmas sorgen. Denkbar ist auch, sonstige aus dem Stand der Technik bekannte Füllmaterialien, z. B. zähflüssige Embolisate wie Onyx, durch die Einführhilfe in das Aneurysma einzubringen.

Ein Vorteil der zusätzlichen Verfüllung mit weiteren Okklusionsmitteln oder - materialien ist darin zu sehen, dass ein Zusammendrücken ("Kompaktieren") des Implantats durch äußere Kräfte verhindert wird. In der Praxis kann es vorkommen, dass das Aneurysma von außen auf das eingebrachte, innen hohle Implantat einwirkt, sodass dieses komprimiert wird und nicht mehr in der Lage ist, das Aneurysma vollständig auszufüllen, oder partiell aus dem Aneurysma herausgedrückt wird, wodurch eine Behinderung des Blutflusses im Stammblutgefäß herbeigeführt würde. Dies wird durch das zusätzliche Verfüllen des Implantatinneren mit Okklusionsmitteln oder -materialien, die durch die rohr- oder schlauchförmige Einführhilfe eingebracht werden, verhindert.

Darüber hinaus können zusätzlich eingebrachte Okklusionsmittel Zwischenräume zwischen dem Äußeren des Implantats und der Innenwandung des Aneurysmas ausfüllen. Dies ist besonders bei unregelmäßig geformten Aneurysmen von Bedeutung. Damit die zusätzlich eingebrachten Okklusionsmittel, insbesondere herkömmliche Okklusionscoils, in den Raum zwischen Implantatäußerem und Aneurysmainnenwandung gelangen können, sollte das distale Segment oder der distale Bereich des Grundkörpers nicht von einer Membran abgedeckt sein, vielmehr sollte sich die Membran, sofern vorhanden, auf das proximale Segment bzw. den proximalen Bereich beschränken. Besonders vorteilhaft ist, wenn die rohr- oder schlauchförmige Einführhilfe bei expandiertem Grundkörper in diesem in etwa mittig endet, damit die durch die Einführhilfe eingebrachten Okklusionsmittel von dort nach außen wandern und durch die Zwischenräume zwischen den Streben in den Bereich zwischen Implantat und Aneurysma gelangen können.

Wenn die Einführhilfe ein inneres Lumen hat, wird die Einbringung von zusätzlichen Okklusionsmittel oder Okklusionsmaterial deutlich vereinfacht. Bei Implantaten, die mit Membranen versehen sind, stellt sich nämlich die Frage der Sondierbarkeit, d. h. wie zusätzliche Okklusionsmittel oder -materialien in das Aneurysma eingebracht werden können, um eine möglichst vollständige Abdichtung gegen das Einströmen von Blut aus dem Stammblutgefäß zu erreichen. Zum nachträglichen Einbringen muss ggf. ein weiterer Katheter zugeführt und die Membran des Implantats durchstoßen werden. Dies wird erfindungsgemäß deutlich vereinfacht, denn die Einbringung von Okklusionsmittel oder -materialien kann bereits bei der Implantation des Implantats erfolgen. Vorzugsweise wird dabei das Implantat zunächst im Aneurysma expandiert, jedoch noch nicht abgelöst. Anschließend werden Okklusionsmittel durch die rohroder schlauchförmige Einführhilfe eingebracht. Schließlich erfolgt die Ablösung und damit die endgültige Freisetzung des Implantats und Einführhilfe und Mikrokatheter können in proximaler Richtung zurückgezogen werden.

Eine Einführhilfe mit innerem Lumen kann auf unterschiedliche Art und Weise zur Verfügung gestellt werden, wobei eine ausreichende Flexibilität gegeben sein sollte, damit die Einführhilfe auch englumigen Blutgefäßen folgen kann, wie sie insbesondere intrakraniell vorkommen. Es kann sich beispielsweise um eine katheterförmige Einführhilfe handeln, d. h. die Einführhilfe kann aus einem Geflecht aus Metall und/oder Kunststoff bestehen. Die Einführhilfe kann jedoch auch als schlauchartiger Hypotube ausgeführt werden. Der Schlauch kann beispielsweise aus einem biegsamen Polymermaterial oder einer Metallhelix aufgebaut sein, wobei im Falle der Metallhelix auch mehrere Lagen möglich sind, typischerweise max. 4 Lagen, vorzugsweise 2 Lagen. Die Lagen bilden somit einen Hohlstrang, aufgebaut aus einer oder mehreren Spiralen. Im Falle mehrerer Lagen von Metallhelices ist der Helixverlauf zweier angrenzender Lagen vorzugsweise gegenläufig, d. h. über einer linksläufigen Helix liegt eine rechtsläufige Helix oder umgekehrt. Die Helices sind somit gegenläufig verdrillt. Das Vorsehen von mehreren gegenläufigen Lagen ist sinnvoll, damit über den Schlauch auch Torsionsbewegungen von proximal nach distal weitergegeben werden können.

Im Falle der Fertigung des Schlauchs aus einem Polymermaterial können Schlitze in den Schlauch eingebracht werden, um die Flexibilität des Schlauchs weiter zu erhöhen.

Sowohl bei einer draht- als auch bei einer rohr- oder schlauchförmigen Einführhilfe ist es von Vorteil, wenn die Flexibilität von proximal nach distal zunimmt. Auf diese Weise wird gewährleistet, dass auf der einen Seite die Einführhilfe im proximalen Bereich ausreichend steif ist, dass ein Vorschub und auch eine Übertragung von Torsionskräften möglich ist, auf der anderen Seite jedoch die Einführhilfe im distalen Bereich so flexibel ist, dass sie auch engen Blutgefäßen folgen kann. Beispielsweise kann der Durchmesser von proximal nach distal abnehmen, wobei die Abnahme gleichmäßig oder in ein oder mehreren Stufen erfolgen kann.

Möglich ist auch der Aufbau der Einführhilfe aus mehreren Komponenten. Insbesondere kann im Falle einer rohr- oder schlauchförmigen Einführhilfe der proximale Bereich als schlauchförmiger Hypotube und der distale Bereich als flexibler Katheter ausgebildet werden.

Unabhängig von der Art, in der die Einführhilfe aufgebaut ist, wird die Einführhilfe durch einen Mikrokatheter an die Zielposition gebracht. Dies gilt auch, wenn die Einführhilfe selbst katheterartig aufgebaut ist. Sinnvoll ist darüber hinaus eine äußere, die Reibung vermindernde Beschichtung der Einführhilfe, beispielsweise mit PTFE (Polytetrafluorethylen, Teflon).

Die Ablösung des Implantats von der Einführhilfe ist elektrolytisch, thermisch, mechanisch oder chemisch möglich. Bei der elektrolytischen Ablösung wird die Ablösestelle elektrolytisch durch Anlegen einer Spannung korrodiert, sodass sich das Implantat von der Einführhilfe löst. Um eine anodische Oxidation des Implantats zu vermeiden, sollte dieses von der Ablösestelle und der Einführhilfe elektrisch isoliert sein. Die elektrolytische Ablösung von Implantaten ist aus dem Stand der Technik hinlänglich bekannt, gerade auch für Coils zum Verschließen von Aneurysmen. Entsprechende Ablösestellen sind z. B. in der WO 2011/147567 A1 beschrieben. Das Prinzip basiert darauf, dass bei Anlegen einer Spannung eine hierfür vorgesehene Ablösestelle aus einem geeigneten Material, insbesondere Metall, in der Regel durch anodische Oxidation eine zumindest so weitgehende Auflösung erfährt, dass die distal der entsprechenden Ablösestelle gelegenen Bereiche des Implantats freigesetzt werden. Die Ablösestelle kann beispielsweise aus Edelstahl, Magnesium, Magnesiumlegierungen oder einer Cobalt-Chrom-Legierung gefertigt sein. Eine besonders bevorzugte Magnesiumlegierung ist Resoloy^{®}, welche von der Firma MeKo aus Sarstedt/Deutschland entwickelt wurde (vgl. WO 2013/024125 A1). Es handelt sich um eine Legierung aus Magnesium und u. a. Lanthanoiden, insbesondere Dysprosium. Vorteilhaft bei der Verwendung von Magnesium und Magnesiumlegierungen ist auch, dass ein Verbleib von Magnesiumresten im Körper physiologisch unproblematisch ist.

Die Auflösung der Ablösestelle erfolgt durch Anlegen einer elektrischen Spannung. Dabei kann es sich sowohl um Wechselstrom als auch um Gleichstrom handeln, wobei eine geringe Stromstärke (< 3 mA) ausreicht. Die Ablösestelle stellt dabei in der Regel die Anode dar, an der die Oxidation und Auflösung des Metalls stattfindet. Wichtig ist, dass die Ablösestelle elektrisch leitend, insbesondere über die Einführhilfe, mit einer Spannungsquelle verbunden ist. Die Einführhilfe muss in diesem Fall auch selbst elektrisch leitfähig ausgebildet sein. Da der sich einstellende Korrosionsstrom von der Fläche der Kathode gesteuert wird, sollte die Fläche der Kathode deutlich größer gewählt werden als die Fläche der Anode. In gewissem Maße lässt sich die Auflösungsgeschwindigkeit der Ablösestelle durch Einstellung der Kathodenfläche im Verhältnis zur Anodenfläche steuern. Die Erfindung betrifft entsprechend auch eine Vorrichtung, die eine Spannungsquelle sowie ggf. eine auf der Körperoberfläche platzierbare Elektrode umfasst.

Insbesondere kann die Ablösestelle in Form eines Ablöseelements vorliegen, das außen an der Einführhilfe angeordnet und mit dem proximalen Ende des Grundkörpers verbunden ist. Durch Anlegen der elektrischen Spannung wird die Verbindung zwischen Ablöseelement und Grundkörper so weitgehend korrodiert, dass eine Ablösung und Freisetzung des Implantats erfolgt. Vorzugsweise ist das Ablöseelement ringförmig um die Einführhilfe angeordnet. So kann das Ablöseelement eine Scheibe mit mittigem Loch zur Durchführung der Einführhilfe bilden. An das Ablöseelement können ein oder mehrere Ablösedrähte angebracht sein, die zweckmäßigerweise eine Isolierung tragen, um die elektrische Spannung möglichst konzentriert an der Ablösestelle anliegen zu lassen. Der oder die Ablösedrahte können außerhalb der Einführhilfe oder auch durch das Innere der Einführhilfe verlaufen. Bei Fertigung der Einführhilfe aus Metall, insbesondere im Falle einer katheterartigen Einführhilfe aus einem Metallgeflecht, kann der Strom auch hierüber zugeführt werden.

Wie bereits erwähnt, ist es von Vorteil, wenn die Einführhilfe in den Grundkörper im expandierten Zustand hineinragt. Insbesondere kann die rohr- oder schlauchförmige Einführhilfe nach distal über die Ablösestelle hinausragen. Bei der oben beschriebenen Ausführungsform mit einem die Einführhilfe ringförmig umgebenden Ablöseelement liegt somit das Ablöseelement weiter proximal als das distale Ende der Einführhilfe. Entsprechend kann man zunächst den Grundkörper in das Aneurysma einbringen und durch Zurückziehen des Mikrokatheters zur Expansion bringen. Über die in den Grundkörper hineinragende Einführhilfe können sodann zusätzliche Okklusionsmittel, insbesondere Coils, in das Innere des Grundkörpers eingebracht werden. Sobald dieser Prozess abgeschlossen ist, erfolgt eine Ablösung des Implantats und schließlich der Rückzug und die Entfernung von Mikrokatheter und Einführhilfe aus dem Blutgefäßsystem.

Bei einer mechanischen Ablösung besteht typischerweise ein Form-, Kraft- oder Reibschluss, der bei der Freisetzung des Implantats aufgehoben wird, sodass sich das Implantat von der Einführhilfe löst. Eine Möglichkeit besteht darin, auf der Außenseite der Einführhilfe im distalen Bereich radiale Vorsprünge vorzusehen, wobei der distale Bereich in das Implantat hineinragt. Außen um die Einführhilfe liegt der Mikrokatheter. Auf diese Weise wird eine reibschlüssige Verbindung zwischen der Einführhilfe, dem proximalen Ende des Implantats und dem Mikrokatheter herbeigeführt. Wenn der Mikrokatheter relativ zum Implantat und zur Einführhilfe in proximaler Richtung zurückgezogen wird, entfällt die durch den Mikrokatheter herbeigeführte äußere Einschnürung und das proximale Ende des Implantats kann radial expandieren und sich von den Vorsprüngen lösen, d. h. die reibschlüssige Verbindung wird aufgelöst.

Unter distalem Bereich der Einführhilfe wird in diesem Zusammenhang ein weit distal liegender Bereich verstanden, der mit dem Implantat interagiert, es muss sich jedoch nicht um das distale Ende der Einführhilfe handeln, welches häufig noch weiter in das Implantat hineinführt, um eine Platzierung von Okklusionsmitteln an dieser Stelle möglich zu machen. Radiale Vorsprünge sind solche, die in radialer Richtung, d. h. orthogonal zur Längsachse der Einführhilfe, nach außen vorstehen.

Der das proximale Ende an der Expansion hindernde äußere Zwang muss nicht in jedem Fall durch den Mikrokatheter herbeigeführt werden, möglich ist auch das Vorsehen einer zusätzlichen, das distale Ende der Einführhilfe umgebenden Umhüllung, die das proximale Ende des Implantats auf die Einführhilfe presst. Zur Freisetzung muss selbstverständlich auch diese Umhüllung relativ zu Einführhilfe und Implantat in proximaler Richtung rückziehbar sein.

Die radialen Vorsprünge können aus einem elastischen Material gefertigt sein, insbesondere einem Elastomermaterial. Das proximale Ende des Implantats wird durch den Mikrokatheter oder die Umhüllung auf die Einführhilfe gepresst und quasi eingeklemmt, sodass eine reibschlüssige Festlegung vorliegt. Die Vorsprünge können insbesondere als Pads geformt sein. Die Pads können die Einführhilfe ringförmig umgeben, wobei zur Herbeiführung einer besonders sicheren Festlegung mehrere radial verlaufende Ringe oder eine Helix vorgesehen sein können.

Auch das proximale Ende des Implantats kann Verdickungen aufweisen, die z. B. zwischen den radialen Vorsprüngen der Einführhilfe gehalten werden. Die Verdickungen können bspw. kugelförmig sein oder eine ähnliche geometrische Form aufweisen. In diesem Fall handelt es sich bei der Festlegung des Implantats an der Einführhilfe um eine Kombination aus Reibschluss und Formschluss.

Um die Ablösung für den behandelnden Arzt zu visualisieren, sind röntgendichte Markierungen im Bereich der Ankopplung des Implantats an die Einführhilfe von Vorteil. Beispielsweise können in diesem Bereich die Einführhilfe, das proximale Ende des Implantats und/oder der Mikrokatheter bzw. eine die Einführhilfe umgebende Umhüllung mit röntgendichten Markierungen versehen sein, deren Bewegung, insbesondere deren Bewegung relativ zueinander durch Röntgen beobachtet werden kann.

Auch eine rein formschlüssige Festlegung ist möglich. So können am proximalen Ende des Implantats Ablöseelemente angeordnet sein, die formschlüssig in hierfür vorgesehene Ausnehmungen in der rohr- oder schlauchförmigen Einführhilfe eingreifen. Da die Einführhilfe vom Mikrokatheter oder einer zusätzlichen Umhüllung umgeben ist, sind die Ablöseelemente daran gehindert, sich radial aufzuweiten und aus den Ausnehmungen auszutreten. Auf diese Weise werden die Ablöseelemente sicher in den Ausnehmungen gehalten, solange kein Rückzug des Mikrokatheters bzw. der Umhüllung in proximaler Richtung erfolgt. Erst wenn Mikrokatheter/Umhüllung nach proximal bewegt werden und die Ausnehmungen nicht mehr abdecken, können die Ablöseelemente aus den Ausnehmungen heraustreten, wodurch das Implantat abgelöst und endgültig freigesetzt wird. Die Ausnehmungen in der Einführhilfe können die gesamte Wandung der Einführhilfe erfassen oder lediglich als Vertiefungen in die Wandung eingelassen sein.

Gemäß einer weiteren Variante erstrecken sich am proximalen Ende des Implantats ein oder mehrere Ablöseelemente in proximaler Richtung, die von einem oder mehreren an der Einführhilfe angeordneten Halteelementen formschlüssig gehalten werden. Das Halteelement ist aus einem Material mit Formgedächtniseigenschaften gefertigt, wobei dem Halteelement eine Sekundärstruktur aufgeprägt ist, die es bestrebt ist einzunehmen, an deren Einnahme es jedoch gehindert ist, solange ein Mikrokatheter oder eine gesonderte Umhüllung das Halteelement umgibt. Insbesondere kann der äußere Zwang das Halteelement an der Annahme der Sekundärstruktur hindern; möglich ist jedoch auch, dass das Haltelement nach Rückzug des Mikrokatheters/der Umhüllung eine Temperaturänderung erfährt, wodurch es seine Sekundärstruktur einnimmt. Bei Annahme der Sekundärstruktur öffnet sich das Halteelement und gibt das Ablöseelement frei, d. h. das Implantat wird abgelöst.

Bei dieser Ausführungsform kann das Ablöseelement beispielsweise kugelförmig sein und das Halteelement das Ablöseelement schalenförmig umschließen. Bei Aufweitung des schalenförmigen Halteelements kann das kugelförmige Ablöseelement austreten. Die Ablösung kann auf Basis eines einzelnen Ablöseelements mit einem einzelnen zusammenwirkenden Halteelement erfolgen, möglich ist jedoch auch, vorzugsweise entlang des Umfangs der Einführhilfe mehrere Halteelemente mit entsprechenden Ablöseelementen zusammenzubringen, um eine gleichmäßige Freisetzung über den gesamten Umfang zu gewährleisten.

Unabhängig davon, ob es sich um eine elektrolytische oder eine mechanische Ablösung handelt, kann das proximale Ende des Implantats, das an der Ablösestelle festgehalten wird, insbesondere in Form von proximalen Drähten vorliegen, deren Verbindung zum Ablöseelement/zur Ablösestelle zwecks Ablösung des Implantats gelöst wird. Insbesondere im Fall der mechanischen Ablösung können an den proximalen Drähten weitere Ablöseelemente angebracht sein, die von der Einführhilfe gehalten werden.

Eine weitere Möglichkeit besteht darin, Ablösestellen als thermische Ablösestellen auszubilden. Bei einer thermischen Ablösestelle kann die Verbindung zwischen in Längsrichtung aneinandergrenzenden Abschnitten des Implantats dadurch aufgehoben werden, dass eine Erwärmung der Ablösestelle erfolgt, woraufhin diese so weich wird oder schmilzt, dass eine Abtrennung erfolgt. Schließlich ist auch eine chemische Ablösung möglich, bei der die Ablösung durch eine chemische Reaktion an der Ablösestelle herbeigeführt wird.

Die unterschiedlichen Arten der Ablösung, beispielsweise elektrolytische und mechanische Ablösung, können auch miteinander kombiniert werden. Hierbei wird zwischen den Einheiten eine mechanische Verbindung, insbesondere über Formschluss hergestellt, die solange besteht, bis ein die mechanische Verbindung aufrechterhaltendes Element elektrolytisch korrodiert wird.

Eine Möglichkeit für eine Kombination aus elektrolytischer und mechanischer Ablösung besteht darin, am distalen Ende der Einführhilfe ein Einführhilfenendstück vorzusehen, das über eine elektrolytisch korrodierbare Ablösestelle verfügt, wobei das Einführhilfenendstück über ein Isolatorelement mit einem Implantatendstück am proximalen Ende des Implantats verbunden ist. Insbesondere kann zwischen dem Einführhilfenendstück, dem Isolatorelement und dem Implantatendstück ein Formschluss bestehen, der eine sichere Verbindung zwischen Einführhilfe und Implantat während des Vorschubs durch den Mikrokatheter gewährleistet. Darüber hinaus kann der Formschluss auch die Übertragbarkeit von Torsionsbewegungen sicherstellen.

Das Einführhilfenendstück ist vorzugsweise mindestens teilweise aus einem Metall gefertigt, das die elektrische Leitfähigkeit zur Ablösestelle sicherstellt, d. h. es kann ein Strom an der Einführhilfe angelegt werden, der bis zur Ablösestelle weitergeleitet wird. Denkbar ist auch das Vorsehen von gesonderten Leitern, die von der eigentlichen Einführhilfe isoliert sind, um einen Strom an der Ablösestelle anlegen zu können. Die Ablösestelle wird normalerweise als Anode geschaltet, um dort die Oxidation des für die Ablösestelle verwendeten Metalls und damit die Auflösung der Ablösestelle herbeizuführen. Als Kathode dient in der Regel eine auf die Körperoberfläche des Patienten aufgebrachte Elektrode; möglich ist jedoch auch das Vorsehen eines weiteren elektrischen Leiters an oder durch die Einführhilfe, der den Stromkreis zur Ablösestelle schließt. Vorzugsweise ist zumindest die Ablösestelle aus einem gut elektrolytisch bzw. galvanisch auflösbarem Metall wie einer Cobalt-Chrom-Legierung gefertigt; möglich sind auch andere nichtrostende Metalllegierungen. Es ist möglich, die Ablösestelle einer Vorkorrosion zu unterziehen, um diese besser auflösbar zu machen.

Auch das Implantatendstück ist zumeist aus Metall gefertigt. Von Vorteil ist die Fertigung aus einem Metall mit Formgedächtniseigenschaften, wie insbesondere Nickel-Titan-Legierungen, oder aus einem Metall wie Magnesium, das sich im Körper verhältnismäßig rasch auflöst, sodass nach einiger Zeit lediglich für die Einbringung, nicht jedoch dauerhaft benötigte Bereiche des Implantats verschwinden. Auch die weiter distal gelegenen Bereiche des Einführhilfenendstücks, die ebenfalls nach Ablösung im Körper verbleiben, können z. B. aus Magnesium gefertigt sein. Unnötige Fremdkörper lösen sich somit auf.

Das Isolatorelement zwischen Einführhilfen- und Implantatendstück sorgt dafür, dass das Implantat von der angelegten elektrischen Spannung isoliert ist, wodurch der Strom auf die Ablösestelle konzentriert wird. Das Isolatorelement ist aus einem elektrisch isolierenden Material gefertigt. Ein Formschluss zwischen den beiden Endstücken wird vorzugsweise durch das Isolatorelement übertragen.

Gemäß einer besonders bevorzugten Ausführungsform weisen sowohl das Einführhilfenendstück als auch das Implantatendstück die Form eines ersten kurzen Rohrelements auf, an das sich in Längsrichtung ein schmaler Verbindungssteg anschließt, der mit einem zweiten kurzen Rohrelement verbunden ist, welches eine Unterbrechung entlang des Umfangs aufweist. Vorzugsweise befindet sich diese Unterbrechung radial betrachtet dem Verbindungssteg gegenüber. Das Isolatorelement ist auf die beiden Endstücke in der Weise abgestimmt, dass die jeweiligen zweiten Rohrelemente mit Unterbrechung jeweils einen Abschnitt des Isolatorelements umgreifen können, wobei die beiden Abschnitte des Isolatorelements in Längsrichtung hintereinander angeordnet sind. Die beiden Abschnitte weisen jeweils Aufnahmen für die zweiten Rohrelemente auf. Als Längsrichtung wird in diesem Zusammenhang die Richtung von proximal nach distal oder andersherum verstanden.

Beim Zusammenfügen von Einführhilfenendstück, Isolatorelement und Implantatendstück ist das zweite Rohrelement des Implantatendstücks proximal des ersten Rohrelements angeordnet, während das zweite Rohrelement des Einführhilfenendstücks distal des ersten Rohrelements angeordnet ist. Das zweite Rohrelement des Implantatendstücks greift in die Aufnahme des weiter proximal angeordneten Abschnitts des Isolatorelements ein, das zweite Rohrelement des Einführhilfenendstücks hingegen in die Aufnahme des weiter distal angeordneten Abschnitts des Isolatorelements. Einführhilfenendstück und Implantatendstück greifen daher letztlich ineinander ein, allerdings stets mit einer Zwischenlage, die vom Isolatorelement gebildet wird. In der zusammengefügten Konfiguration liegt das zweite Rohrelement des Implantatendstücks proximal des zweiten Rohrelements des Einführhilfenendstücks. Die Unterbrechungen in den zweiten Rohrelementen der Endstücke befinden sich in der Regel in radial gegenüberliegenden Positionen.

Zweckmäßigerweise im Bereich des schmalen Verbindungsstegs des Einführhilfenendstücks ist die Ablösestelle angeordnet, beispielsweise dort, wo der Verbindungssteg des Einführhilfenendstücks mit dem ersten, durchgehenden Rohrelement verbunden ist. Die Ablösestelle ist leicht korrodierbar und löst sich durch Anlegen einer Spannung jedenfalls so weitgehend auf, dass sich Bereiche des Einführhilfenendstücks, z. B. das erste Rohrelement von den weiteren Bereichen des Ablöseelements abtrennen. Als Ablöseelement wird in diesem Zusammenhang die Gesamtheit aus Einführhilfenendstück, Isolatorelement und Implantatendstück verstanden. Das Implantat samt Implantendstück, Isolatorelement und den weiter distal gelegenen Bereichen des Einführhilfenendstücks verbleibt somit im Körper, während der proximale Bereich des Einführhilfenendstücks mit der Einführhilfe verbunden bleibt und aus dem Körper entfernt wird.

Die Vorteile des beschriebenen Ablöseelements, das eine Kombination aus mechanischer Verbindung über Formschluss mit elektrolytischer Ablösbarkeit darstellt, liegen insbesondere in der mechanischen Sicherheit der Verbindung, die z. B. auch beim Zurückziehen der Einführhilfe in Richtung proximal gegeben ist. Auch nach Ausschieben aus dem bzw. Rückzug des Mikrokatheters ist eine mechanische Verbindung gegeben. Eine Trennung erfolgt erst und gut steuerbar bei Anlegen einer elektrischen Spannung an das Ablöseelement.

Die beschriebenen Ablöseelemente können grundsätzlich auch zum Einbringen beliebiger anderer Implantate verwendet werden. Dies gilt besonders für die zuletzt beschriebene Ausführungsform, die eine Kombination aus elektrolytischer Ablösung und mechanischer Verbindung darstellt und daher besonders sicher ist. Von der Erfindung umfasst sind somit entsprechende Ablösesysteme, auch wenn das Implantat selbst eine Ausgestaltung aufweist, die nicht den in dieser Patentanmeldung beschriebenen Ausgestaltungen entspricht.

Die Ablösestelle kann proximal des Grundkörpers angeordnet sein, bevorzugt ist jedoch wie beschrieben das Vorsehen einer Ablösestelle innerhalb des Grundkörpers bzw. des Implantats, sodass sich die Einführhilfe in das Implantat im expandierten Zustand hinein erstreckt. Zum einen erleichtert dies das Einbringen weiterer Okklusionsmittel, wenn die Einführhilfe rohr- oder schlauchförmig ausgebildet ist, zum anderen können die Streben, die das proximale Segment bzw. den proximalen Bereich ausbilden, an der Ablösestelle zumindest teilweise zusammenlaufen, wodurch im proximalen Segment/Bereich eine leichte Einstülpung herbeigeführt wird. Eine Einstülpung im proximalen oder auch im distalen Segment/Bereich ist mit dem Vorteil verbunden, dass potentiell traumatische Enden der Streben vermieden werden bzw. nicht mit der Aneurysmawandung in Kontakt kommen können, weil sie in das Innere des Grundkörpers verlegt sind.

Unter einer Abdeckung mit der Membran wird jedwedes Versehen der entsprechenden Streben bzw. Segmente mit einer Membran verstanden, unabhängig davon, ob die Membran außen oder innen auf den Streben/Segmenten aufgebracht ist oder die Streben/Segmente in die Membran eingebettet sind. Letzteres ist bevorzugt.

Sofern das Implantat über eine Membran verfügt, kann es alleine, d. h. ohne zusätzliche Okklusionsmittel wie Coils o. ä., das Aneurysma vom Blutstrom abkoppeln und verschließen. Die Membranen weisen eine ausreichende Elastizität auf, sodass das Ein- und Entfalten des Implantats nicht behindert und die Membranen dabei nicht beschädigt werden. Besonders vorteilhaft ist eine Membran im proximalen Segment oder Bereich des Implantats, weil auf diese Weise eine Abschottung gegenüber dem Stammblutgefäß herbeigeführt wird. Häufig reicht es, lediglich ein entsprechendes Implantat in das Aneurysma einzuführen, es ist jedoch auch möglich, mehrere erfindungsgemäße Implantate zu verwenden. Des Weiteren können, wie oben beschrieben, Okklusionsmittel oder Okklusionsmaterial in das Implantat eingebracht werden. In diesem Zusammenhang ist eine Einführhilfe mit innerem Lumen besonders von Vorteil. Es kann auch ein Stent oder Flow Diverter im Stammblutgefäß gesetzt werden.

Es sind jedoch auch Ausführungsformen der Erfindung denkbar, bei denen der Grundkörper nicht mit einer Membran versehen ist. In diesem Fall dient das Implantat normalerweise als Stützstruktur zum Zurückhalten weiterer Implantate wie Coils, erfindungsgemäßen Implantaten geringerer Größe mit oder ohne Membran oder auch anderen in der Einleitung beschriebenen Okklusionsmitteln. Denkbar ist auch, das erfindungsgemäße Implantat zusammen mit anderen aus dem Stand der Technik bekannten Füllmaterialien, z. B. zähflüssigen Embolisaten wie Onyx zu verwenden. Sofern weitere Okklusionsmittel oder -materialien nicht bereits bei der Implantation des Implantats eingebracht werden, erfolgt normalerweise zunächst das Einsetzen des Implantats in das Aneurysma, anschließend kann ein Mikrokatheter zwischen den das Implantat ausbildenden Streben eingeführt und Okklusions- bzw. Embolisationsmittel durch den Mikrokatheter in das Aneurysma eingebracht werden.

Soweit im Rahmen dieser Erfindung von Membranen im Plural gesprochen wird, sei hiermit klargestellt, dass zwischen den einzelnen Membranen keine Trennung vorliegen muss, vielmehr können die einzelnen Membranen durchaus ineinander übergehen und somit eine Gesamtmembran ausbilden. Umgekehrt kann sich eine Membran, beispielsweise die das proximale Segment oder den proximalen Bereich abdeckende Membran, auch aus mehreren Einzelmembranen zusammensetzen, die beispielsweise jeweils eine Masche/Schlaufe im proximalen Segment ausfüllen.

Als Membran wird erfindungsgemäß eine dünne Struktur mit flächiger Ausdehnung verstanden, unabhängig davon, ob diese für Flüssigkeiten durchlässig, undurchlässig oder teildurchlässig ist. Zur Erfüllung des Zwecks der Behandlung eines Aneurysmas sind allerdings Membranen bevorzugt, die für Flüssigkeiten wie Blut vollständig oder zumindest weitgehend undurchlässig sind. Darüber hinaus kann eine Membran auch mit Poren versehen sein, durch die weitere Okklusionsmittel eingebracht werden können. Eine weitere Möglichkeit besteht darin, die Membran so auszubilden, dass sie mit einem Mikrokatheter zur Einführung weiterer Okklusionsmittel oder auch mit den Okklusionsmitteln selbst durchstoßen werden kann.

Die Membranen können aus Polymerfasern oder -folien aufgebaut sein. Vorzugsweise werden die Membranen durch Elektrospinnen erzeugt. Dabei werden normalerweise die Streben in die Membran eingebettet. Dies kann dadurch erreicht werden, dass die Streben, insbesondere die Rahmenstreben des proximalen und/oder distalen Segments, mit Fasern umsponnen bzw. umflochten werden.

Beim Elektrospinnen werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3.000 nm. Durch Elektrospinnen gewonnene Membranen sind sehr gleichmäßig ausgebildet. Die Membran ist zäh und mechanisch belastbar und kann mechanisch durchstoßen werden, ohne dass die Öffnung zum Ansatzpunkt für weitergehende Risse wird. Ein weiterer Vorteil von elektrogesponnenen Membranen ist darin zu sehen, dass eine große Kontaktoberfläche zum Blut hergestellt wird. Die Dicke der Fibrillen wie auch der Grad der Porosität kann durch Auswahl der Verfahrensparameter gesteuert werden. Im Zusammenhang mit der Schaffung der Membran und den dafür geeigneten Materialien wird insbesondere auf die WO 2008/049386 A1, die DE 28 06 030 A1 und die darin genannte Literatur hingewiesen.

Statt durch Elektrospinnen können die Membranen auch über einen Tauch- oder Sprühprozess wie Spraycoaten hergestellt werden. Wichtig ist hinsichtlich des Materials der Membranen, dass diese durch die mechanische Beanspruchung beim Einziehen in einen Mikrokatheter, Ausbringen, Entfalten etc. nicht beschädigt werden. Die Membranen sollten daher eine ausreichende Elastizität aufweisen.

Die Membranen können aus einem Polymermaterial wie Polytetrafluorethylen, Polyester, Polyamiden, Polyurethanen oder Polyolefinen hergestellt sein. Besonders bevorzugt sind Polycarbonaturethane (PCU), insbesondere elektrogesponnene Polycarbonaturethane. Wünschenswert ist insbesondere eine integrale Verbindung der Membranen mit den Streben/Segmenten. Eine solche integrale Verbindung kann durch kovalente Bindungen zwischen den Membranen und den Streben/Segmenten erreicht werden. Die Ausbildung von kovalenten Bindungen wird durch eine Silanisierung der Streben/Segmente gefördert, d. h. durch eine chemische Anbindung von Silicium-, insbesondere Silanverbindungen an zumindest Teilen der Oberfläche der Streben/Segmente. Silicium- und Silanverbindungen binden an Oberflächen beispielsweise an Hydroxy- und Carboxy-Gruppen. Neben der Silanisierung sind grundsätzlich auch andere Methoden der Haftvermittlung zwischen Streben/Segmenten und Membranen denkbar.

Unter einer Silanverbindung sind in diesem Zusammenhang all jene Verbindungen zu verstehen, die der allgemeinen Formel RₘSiXₙ folgen (m, n = 0-4) wobei R für organische Reste, insbesondere Alkyl-, Alkenyl- oder Arylgruppen, und X für hydrolysierbare Gruppen, insbesondere OR, OH oder Halogen mit R = Alkyl, Alkenyl oder Aryl steht. Insbesondere kann das Silan die allgemeine Formel RSiX₃ haben. Darüber hinaus gehören entsprechende Verbindungen mit mehreren Siliciumatomen zu den Silanverbindungen. Insbesondere Silan-Derivate in Form von siciliumorganischen Verbindungen werden in diesem Zusammenhang als Silanverbindungen aufgefasst.

In die Membranen eingebettet oder auf die Membranen aufgebracht sein können zusätzliche die Thrombogenisierung oder die Endothelbildung fördernde Stoffe. Die Thrombogenisierung fördernde Stoffe sind deshalb von Vorteil, weil sie die Ausbildung eines Thrombus innerhalb des Aneurysmas unterstützen, welche für einen dauerhaften Verschluss des Aneurysmas sorgt. Ein Beispiel sind Nylonfilamente. Da Aneurysmen auf degenerativen Gefäßwanderkrankungen beruhen, insbesondere der Arteriosklerose, können auch die Förderung der Endothelbildung und die Behebung von Funktionsstörungen des Endothels positive Wirkungen haben. Dies gilt insbesondere für den Bereich, wo das Aneurysma Kontakt zum Blutstrom im eigentlichen Blutgefäß (Stammblutgefäß) hat. Vorzugsweise sind die Thrombogenisierung fördernde Stoffe auf der Innenseite der Membran, die Endothelbildung fördernde Stoffe hingegen auf der Außenseite der Membran aufgebracht, wobei unter Außenseite die im implantierten Zustand der Gefäßwandung zugewandte Seite einer Membran und unter Innenseite die zum Aneurysmainneren weisende Seite einer Membran verstanden wird. Beispiele für die Thrombogenisierung fördernde Stoffe sind Kollagene, während z. B. Hyaluronsäure, Statine (3-Hydroxy-3-Methylglutaryl-Coenzym-A-Reduktase-Inhibitoren) und andere Polymere die Besiedlung mit Endothelzellen fördern können. Als Polymere eignen sich insbesondere Polysaccharide, besonders Glykosaminoglykane, die in der Lage sind, die Glykokalyx zu imitieren. Ein anderes verwendbares Material ist POSS-PCU (polyhedral oligomeric silsesquioxane poly(carbonate-urea) urethane). Es handelt sich um ein Nanokomposit, das u. a. als Gerüst für künstliche Organe sowie als Coating für medizinische Vorrichtungen beschrieben wurde (Tan et al., Crit Rev. Biomed Eng. 2013; 41(6): 495-513). Möglich ist auch die Verwendung von POSS-PCL (polyhedral oligomeric silsesquioxane poly(caprolactone-urea) urethane). Sowohl für POSS-PCU als auch für POSS-PCL gilt, dass insbesondere auch funktionalisierte Derivate dieser Nanokomposite verwendet werden können. Dies gilt insbesondere für solche Derivate, die sich durch Verknüpfung mit Polyacrylsäure (Poly-AA) erhalten lassen. POSS-PCU- bzw. POSS-PCL-Nanokompositpolymere sind für die unmittelbare Immobilisierung auf der Oberfläche eines Implantats nur schlecht geeignet ist, weshalb sich als vorteilhaft herausgestellt hat, Polymere wie Polyacrylsäure (Poly-AA) mit dem Nanokomposit zu verbinden. Dies kann beispielsweise durch Plasmapolymerisation von Acrylsäure geschehen. Eine auf diese Weise erhaltene Poly-AA-g-POSS-PCU-Oberfläche fördert die Bindung von Kollagen (insbesondere Kollagen Typ 1) und somit die Endothelbildung (vgl. Solouk et al., Mater Sci Eng C Mater Biol Appl. 2015; 46: 400-408). Einige Zusätze, beispielsweise Kollagen oder Hyaluronsäure, sind auch deshalb von Vorteil, weil sie die Reibung gegenüber der Innenseite eines Katheters während des Vorschubs und die Biokompatibilität des Implantats verbessern können. Allgemein können biofunktionelle oder bioaktive Beschichtungen auf der Membran vorhanden sein.

Vorzugsweise weist der Grundkörper im expandierten Zustand näherungsweise eine Kugelform, Ellipsoidform, Ovoidform oder die Form eines Zylinders mit konvex nach außen gewölbten Grundflächen auf. Insbesondere im Fall der zweiten Ausführungsform kann die Form des Grundkörpers im expandierten Zustand auch als geschlossene Tulpenblüte beschrieben werden. Dabei wird unter den angegebenen geometrischen Form nicht lediglich die exakte Form verstanden, sondern auch eine Form, die der jeweiligen geometrischen Form zumindest nahekommt. Eine Kugelform ist grundsätzlich gut geeignet, ein regelmäßig geformtes Aneurysma auszufüllen. Wenn jedoch das Implantat in Längsrichtung gestaucht oder auseinandergezogen wird, ergibt sich eine Form, die eher als Ellipsoidform oder als Form eines Zylinders mit konvex nach außen gewölbten Grundflächen bezeichnet werden kann, je nachdem, wie stark die Abrundungen/Abschrägungen der proximalen und distalen Kuppeln und die Streckung/Stauchung des Grundkörpers in Längsrichtung ausfallen. Als Grundflächen des Zylinders werden in diesem Zusammenhang die Stirnseiten angesehen. Der Durchmesser bzw. die Länge des Grundkörpers im expandierten Zustand beträgt typischerweise 4 bis 25 mm, je nach Einsatzzweck. Ein solcher Durchmesser ist ausreichend, um typische Aneurysmen auszufüllen, insbesondere solche, die im intrakraniellen Bereich auftreten. Der tatsächlich innerhalb des Aneurysmas eingenommene Durchmesser kann variieren, wodurch das Implantat zur Behandlung von Aneurysmen unterschiedlicher Größe und mit unterschiedlicher Aneurysmahalsgröße einsetzbar ist.

Sinnvollerweise verfügt das Implantat über ein oder mehrere röntgendichte Markierungen, um dem behandelnden Arzt eine Visualisierung zu ermöglichen. Dies können beispielsweise eine Helix, Spirale oder ein Niet aus einem röntgendichten Material sein, die am Implantat festgelegt sind. Sinnvoll sind auch röntgendichte Markierungen am Mikrokatheter, wie oben beschrieben. Die röntgendichten Markierungen können z. B. aus Platin, Palladium, Platin-Iridium, Tantal, Gold, Wolfram oder anderen röntgendichten Metallen sein. Möglich ist auch, das Implantat, insbesondere die Streben des Grundkörpers mit einer Beschichtung aus einem röntgendichten Material zu versehen, beispielsweise mit einer Goldbeschichtung. Diese kann z. B. eine Stärke von 1 bis 6 µm aufweisen. Die Beschichtung mit einem röntgendichten Material muss nicht den gesamten Grundkörper umfassen. Auch beim Vorsehen einer röntgendichten Beschichtung kann es allerdings sinnvoll sein, zusätzlich einen oder mehrere röntgendichte Marker am Implantat anzubringen, insbesondere am distalen Ende des Implantats.

Eine weitere Möglichkeit, das Implantat röntgensichtbar zu machen, besteht darin, in die Membran röntgendichte Substanzen, beispielsweise Schwermetallsalze wie Bariumsulfat einzubetten. Derartige Stoffe sind z. B. als Kontrastmittel in der Röntgentechnik bekannt.

Eine zusätzliche Option besteht in der Verwendung von Streben aus einem Metall mit Formgedächtniseigenschaften, insbesondere einer entsprechenden Nickel-TitanLegierung, die jedenfalls teilweise einen Platinkern aufweisen. Derartige Streben sind als DFT-Drähte (DFT = drawn filled tubing) bekannt. Auf diese Weise werden die vorteilhaften Eigenschaften von Nickel-Titan einerseits, nämlich die Formgedächtniseigenschaften, mit den vorteilhaften Eigenschaften des Platins andererseits, nämlich der Röntgensichtbarkeit, kombiniert.

Das erfindungsgemäße Implantat eignet sich insbesondere zur Behandlung von intrakraniellen Aneurysmen, denkbar ist jedoch auch die Verwendung für andere Arten von Aneurysmen, beispielsweise Aortenaneurysmen oder peripheren Aneurysmen, wobei die Dimensionen des Implantats entsprechend anzupassen sind.

Neben dem Implantat selbst betrifft die Erfindung auch die Verwendung des Implantats zur Behandlung arteriovenöser Fehlbildungen, insbesondere Aneurysmen. Sämtliche Ausführungen, die zum Implantat selbst gemacht wurden, gelten in entsprechender Weise auch für die Verwendung des Implantats und ein Verfahren zur Anwendung des Implantats.

Die Erfindung wird anhand der Figuren beispielhaft näher erläutert. Es ist darauf hinzuweisen, dass die Figuren bevorzugte Ausführungsvarianten der Erfindung zeigen, die Erfindung ist jedoch nicht darauf beschränkt. Insbesondere umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig. 1: Ein erfindungsgemäßes Implantat gemäß der ersten Ausführungsform in der Seitenansicht in einem Aneurysma;
- Fig. 2: das erfindungsgemäße Implantat aus Fig. 1 in einer Ansicht X - X von distal;
- Fig. 3: ein erfindungsgemäßes Implantat gemäß der ersten Ausführungsform in der Seitenansicht mit einer proximal des Grundkörpers angeordneten Ablösestelle;
- Fig. 4: ein erfindungsgemäßes Implantat gemäß der ersten Ausführungsform in der Seitenansicht mit einer innerhalb des Grundkörpers angeordneten Ablösestelle;
- Fig. 5: zeigt ein in ein Aneurysma eingebrachtes Implantat gemäß der ersten Ausführungsform mit einer das proximale Segment abdeckenden Membran;
- Fig. 6: zeigt ein in ein Aneurysma eingebrachtes Implantat gemäß der ersten Ausführungsform mit einer das proximale und das distale Segment abdeckenden Membran;
- Fig. 7: zeigt ein in ein Aneurysma eingebrachtes Implantat gemäß der ersten Ausführungsform ohne Membran mit proximal des Grundkörpers angeordneter Ablösestelle;
- Fig. 8: zeigt ein in ein Aneurysma eingebrachtes Implantat gemäß der ersten Ausführungsform ohne Membran mit innerhalb des Grundkörpers angeordneter Ablösestelle;
- Fig. 9: zeigt ein in ein Aneurysma eingebrachtes Implantat gemäß der ersten Ausführungsform mit einer das proximale Segment abdeckenden Membran mit innerhalb des Grundkörpers angeordneter Ablösestelle;
- Fig. 10: zeigt ein Implantat gemäß der zweiten Ausführungsform bei der Freisetzung im Aneurysma;
- Fig. 11: zeigt die Freisetzung des Implantats aus Fig. 10 in einem weiter fortgeschrittenen Stadium;
- Fig. 12: zeigt ein Implantat gemäß der zweiten Ausführungsform mit tulpenblütenförmigem Grundkörper;
- Fig. 13: zeigt die Einbringung zusätzlicher Okklusionsmittel in das Implantat aus Fig. 12;
- Fig. 14: zeigt eine elektrolytisch korrodierbare Ablösestelle zur Ablösung des Implantats;
- Fig. 15: zeigt eine weitere elektrolytisch korrodierbare Ablösestelle zur Ablösung des Implantats;
- Fig. 16: zeigt eine mechanisch lösbare, auf Reibschluss basierende Ablösestelle zur Ablösung des Implantats;
- Fig. 17: zeigt die mechanisch lösbare Ablösestelle zur Ablösung des Implantats aus Fig. 16 in einer Vergrößerung;
- Fig. 18: zeigt weitere Beispiele für auf Reibschluss basierende mechanisch lösbare Ablösestellen;
- Fig. 19: zeigt eine Einführhilfe in Form eines flexiblen Schlauchs;
- Fig. 20: zeigt eine mechanisch lösbare, auf Formschluss basierende Ablösestelle zur Ablösung des Implantats;
- Fig. 21: zeigt eine weitere mechanisch lösbare, auf Formschluss basierende Ablösestelle zur Ablösung des Implantats;
- Fig. 22: zeigt ein Implantat mit zweigeteilter Einführhilfe;
- Fig. 23: zeigt ein weiteres Implantat mit zweigeteilter Einführhilfe;
- Fig. 24: zeigt eine weitere Variante für eine auf Formschluss basierende Ablösestelle zur Ablösung des Implantats im geschlossenen Zustand in einer Schrägansicht;
- Fig. 25: zeigt die Ablösestelle aus Fig. 24 im geschlossenen Zustand in der Seitenansicht;
- Fig. 26: zeigt die Ablösestelle aus Fig. 24 im geöffneten Zustand in einer Schrägansicht;
- Fig. 27: zeigt ein Ablöseelement mit formschlüssiger Verbindung zwischen Einführhilfe und Implantat, welches eine elektrolytische Ablösung erlaubt;
- Fig. 28: zeigt die einzelnen Bestandteile des Ablöseelements aus Fig. 27;
- Fig. 29: zeigt das Ablöselement aus Fig. 27 in verschiedenen Perspektiven;
- Fig. 30: zeigt eine Explosionsdarstellung der ineinandergefügten Bestandteile des Ablöseelements aus Fig. 27 und
- Fig. 31: zeigt die Ablösung des Implantats bei Verwendung des Ablöseelements aus Fig. 27.

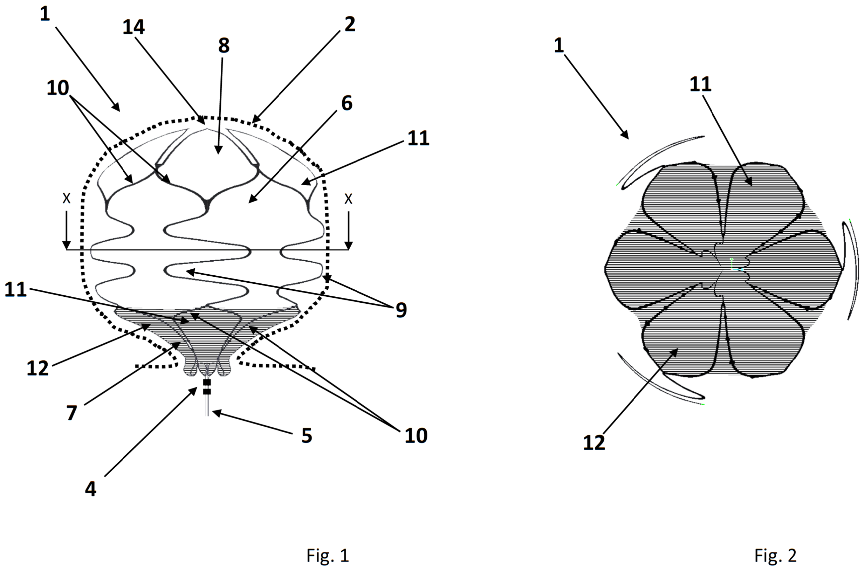

In Figur 1 wird die Erfindung gemäß der ersten Ausführungsform in der Seitenansicht dargestellt. Das Implantat 1 ist in einem gepunktet angedeuteten Aneurysma 2 platziert worden. Das Implantat 1 weist einen Grundkörper 6 auf, der sich aus einem proximalen Segment 7, einem distalen Segment 8 und die beiden Segmente verbindenden Verbindungsstreben 9 zusammensetzt.

Das proximale Segment 7 wird von einer Membran 12 abgedeckt. Sowohl das proximale Segment 7 als auch das distale Segment 8 sind aus Rahmenstreben 10 aufgebaut, die einzelne Maschen oder Schlaufen 11 ausbilden. Im proximalen Segment 7 sind die Rahmenstreben 10 in die Membran 12 eingebettet. Die Verbindungstreben 9 haben einen kurvenlinienförmigen Verlauf, welcher dafür sorgt, dass sich das Implantat 1 sowohl axial als auch radial gut an die jeweiligen Gegebenheiten des Aneurysmas 2 anpassen kann. Diese Flexibilität wird durch einen Bereich 14 im distalen Segment 8 noch weiter erhöht, welcher frei von Rahmenstreben 10 ist.

Im proximalen Bereich ist das Implantat 1 über eine Ablösestelle 4 mit einer Einführhilfe 5 verbunden, bei der es sich um einen Einführdraht handelt. Die Ablösestelle 4 kann beispielsweise elektrolytisch ablösbar ausgebildet sein, sodass nach erfolgter Platzierung des Implantats 1 im Aneurysma 2 eine Ablösung des Implantats 1 erfolgen kann.

In Figur 2 ist gemäß der Schnittansicht X - X das Implantat 1 von distal aus gesehen dargestellt. Man erkennt die einzelnen durch die Rahmenstreben 10 ausgebildeten Schlaufen 11, welche von einer Membran 12 abgedeckt sind. Die Membran 12 hat eine blutflussmodulierende Wirkung und bewirkt, dass das Aneurysma 2 weitgehend vom Blutstrom abgeschnitten wird.

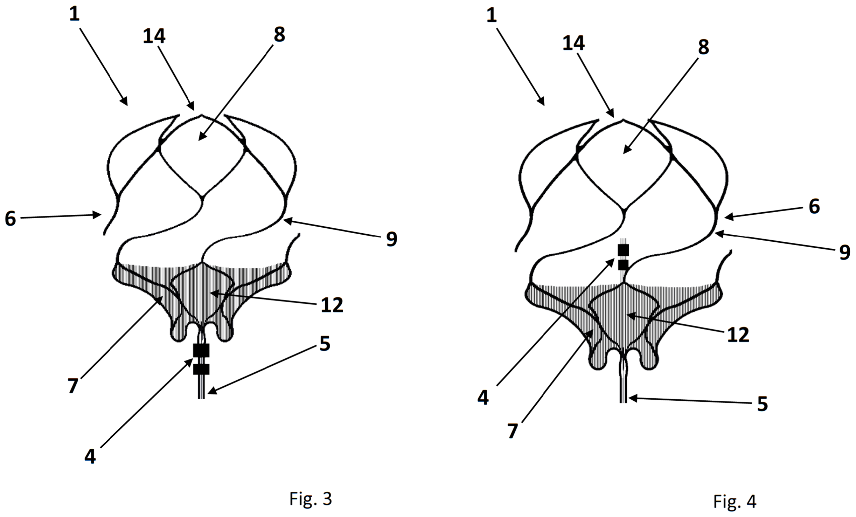

In Figur 3 erkennt man die erste Ausführungsform des erfindungsgemäßen Implantats 1 in der Seitenansicht. Das Implantat 1 weist wiederum ein distales Segment 8 und ein proximales Segment 7 auf, welche durch Verbindungsstreben 9 miteinander verbunden sind. Die Verbindungsstreben 9 verlaufen kurvenlinienförmig. Das proximale Segment 7 ist von einer Membran 12 abgedeckt. Weiter proximal schließt sich die Verbindung des Implantats 1 mit der Einführhilfe 5 über die Ablösestelle 4 an.

Die Darstellung in Figur 4 entspricht weitgehend der aus Figur 3, allerdings ist in diesem Fall die Ablösestelle 4 innerhalb des Grundkörpers 6 vorgesehen. Dies bewirkt, dass auch nach Ablösung des Implantats 1 am proximalen Ende des Implantats 1 keine vorstehenden Drahtspitzen verbleiben.

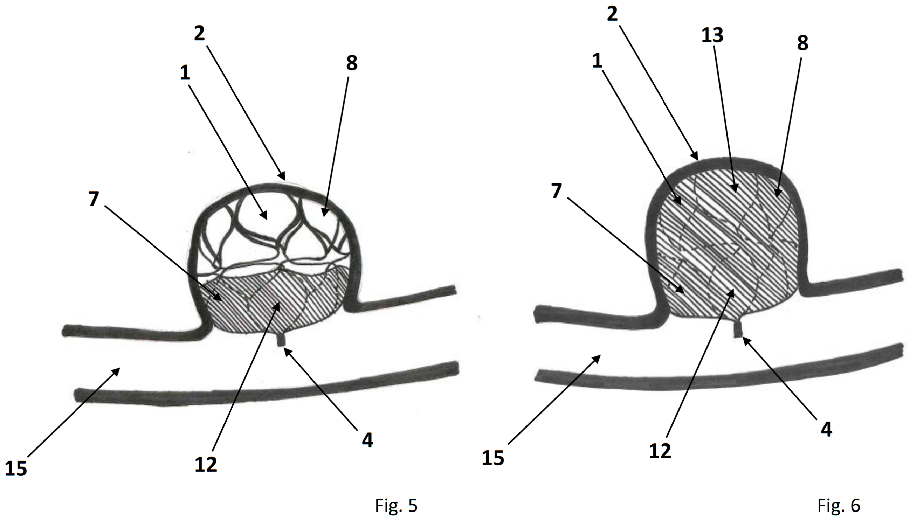

In Figur 5 ist das Implantat 1 vollständig freigesetzt worden. Im vorliegenden Fall handelt es sich um ein Implantat 1, bei dem das proximale Segment 7 von einer Membran 12 abgedeckt ist, das distale Segment 8 hingegen nicht. Die Ablösestelle 4 befindet sich proximal des Grundkörpers 6.

In Figur 6 ist ein entsprechendes Implantat 1 dargestellt, das sich von dem in Figur 5 dargestellten Implantat 1 dadurch unterscheidet, dass hier auch das distale Segment 8 über eine Membran 13 verfügt. Eine weitergehende Abdeckung des Implantats 1 mit einer Membran 13 hat den Vorteil, dass ein Einstrom von Blut in das Aneurysma 2 auch im Falle von Endoleaks weitgehend verhindert wird.

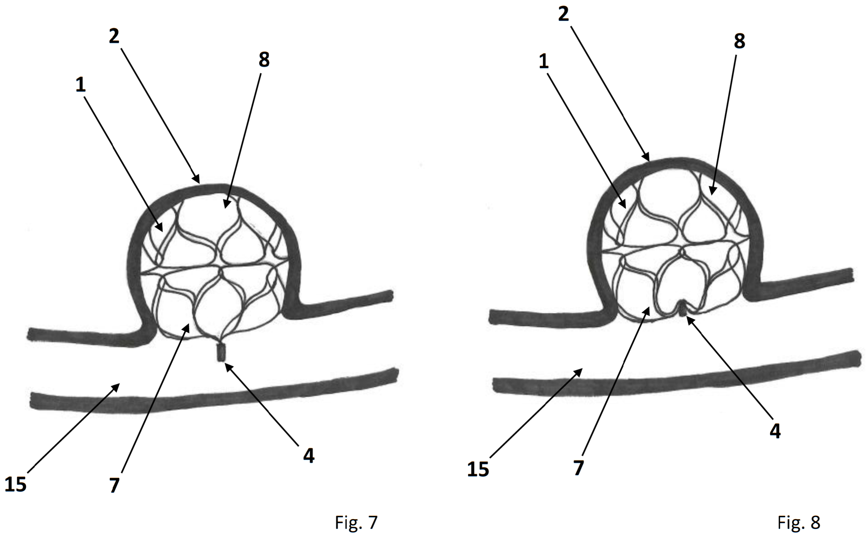

In Figur 7 ist ein entsprechendes Implantat 1 im Aneurysma 2 platziert dargestellt, bei dem auf Membranen verzichtet wurde. Ein solches Implantat 1 dient in erster Linie als Stützstruktur, um zusätzlich eingebrachte Okklusionsmittel im Aneurysma 2 zurückzuhalten.

Figur 8 entspricht weitgehend Figur 7, jedoch ist, anders als in Figur 7, die Ablösestelle 4 innerhalb des Grundkörpers 6 angeordnet. Entsprechend ergibt sich eine zentrale Einstülpung des Grundkörpers 6 nach innen und ein Hineinragen von Teilen des Implantats 1 in das Hauptblutgefäß 15 wird verhindert.

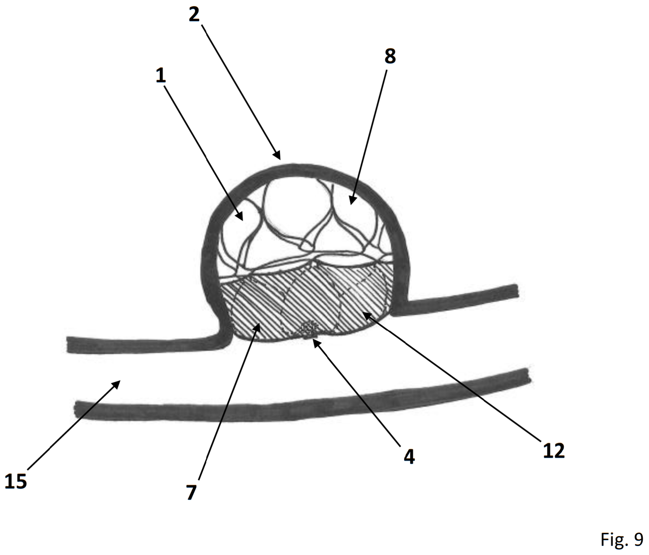

In Figur 9 schließlich ist ein weiteres Implantat 1 mit innerhalb des Grundkörpers 6 angeordneter Ablösestelle 4 gezeigt, bei dem das proximale Segment 7 von einer Membran 12 abgedeckt ist. Das Implantat 1 entspricht somit weitgehend dem aus Figur 5, jedoch mit abweichender Anordnung der Ablösestelle 4.

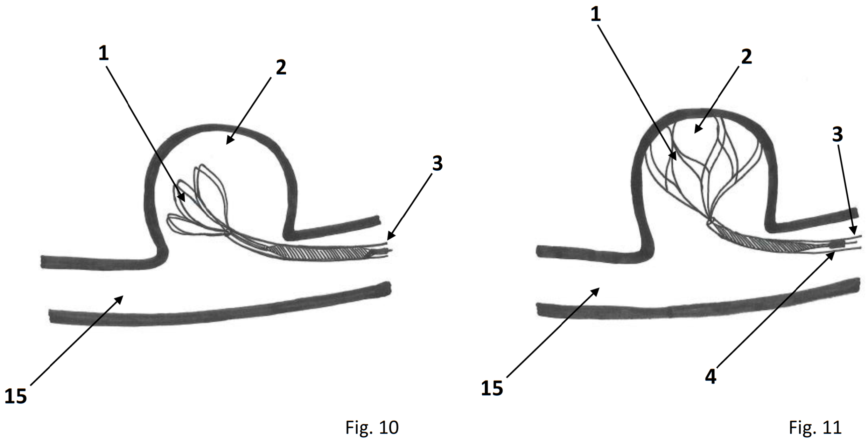

In Figur 10 ist eine zweite Ausführungsform des erfindungsgemäßen Implantats 1 dargestellt, das nach Expansion eine tulpenblütenartige Gestalt annimmt. Das Implantat 1 wird durch einen Mikrokatheter 3 über das Blutgefäß 15 in das Aneurysma 2 eingebracht und durch Vorschub des Implantats 1 bzw. Zurückziehen des Mikrokatheters 3 freigesetzt. Im hier dargestellten Zustand hat sich lediglich das distale Segment 8 des Implantats 1 entfaltet, während sich weiter proximal liegende Bereiche des Implantats 1 noch im Mikrokatheter 3 befinden.

In Figur 11 ist die Freisetzung des Implantats 1 aus Figur 10 weiter fortgeschritten. Man erkennt die Ablösestelle 4, die sich jedoch noch im Mikrokatheter 3 befindet.

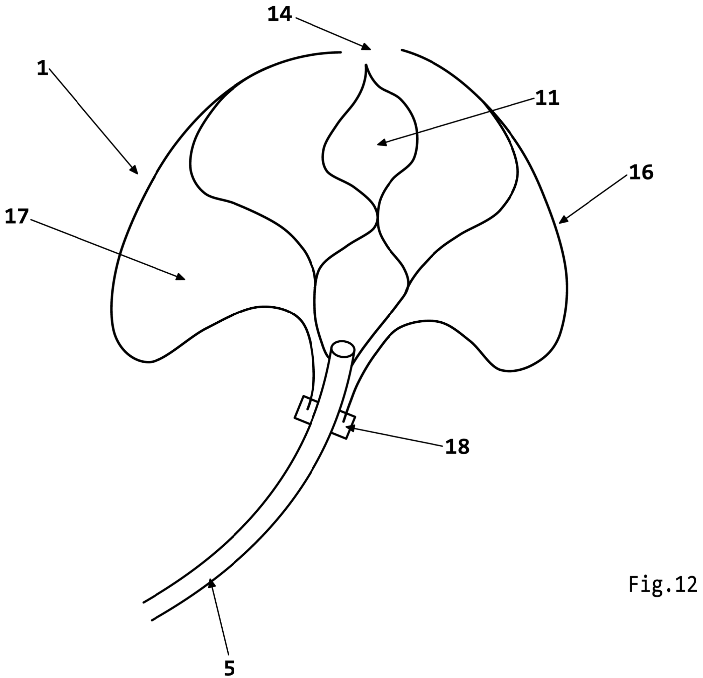

In Figur 12 ist das Implantat gemäß der zweiten Ausführungsform in der Seitenansicht dargestellt. Das Implantat 1 setzt sich aus zum Teil miteinander verbundenen Streben 16 zusammen, zwischen denen sich maschenartige Zwischenräume 11 ausbilden. Insgesamt weist das Implantat 1 mehrere blütenblattförmige Segmente 17 auf, die sich vom proximalen Ende des Implantats 1 ausgehend zunächst nach radial außen und leicht in Richtung proximal erstrecken und anschließend weiter nach distal und radial nach innen verlaufen. Auf diese Weise schmiegen sich die blütenblattförmigen Segmente 17 an die Innenwandung des Aneurysmas 2 an. Am distalen Ende des Implantats 1 verbleibt ein zentraler Bereich 14 ohne Streben 16, d. h. eine Öffnung, die je nach Größe des Aneurysmas 2 mehr oder weniger groß sein kann.

Am proximalen Ende sind einzelne Streben 16 des Implantats 1 mit einem ringförmig um die Einführhilfe 5 angeordneten Ablöseelement 18 verbunden. Die Einführhilfe 5 ist schlauchartig ausgebildet und weist ein inneres Lumen auf, durch welches zusätzliche Okklusionsmittel in das Implantat 1 eingebracht werden können. Durch elektrolytische Korrosion am ringförmigen Ablöseelement 18 lässt sich eine Ablösung des Implantats 1 erreichen. Insbesondere kann das Implantat 1 zunächst in das Aneurysma 2 eingebracht und dort zur Expansion gebracht werden, ohne dass bereits eine Ablösung am Ablöseelement 18 stattgefunden hat. Sodann können durch die Einführhilfe 5 weitere Okklusionsmittel zum Ausfüllen des Innenraums des Implantats 1 eingebracht werden. Wenn dieser Prozess beendet ist, wird schließlich am ringförmigen Ablöseelement 18 ein elektrischer Strom angelegt, um eine Ablösung des Implantats 1 herbeizuführen. Abschließend werden Einführhilfe 5 und der hier nicht dargestellte Mikrokatheter 3 nach proximal zurückgezogen und aus dem Blutgefäßsystem entfernt.

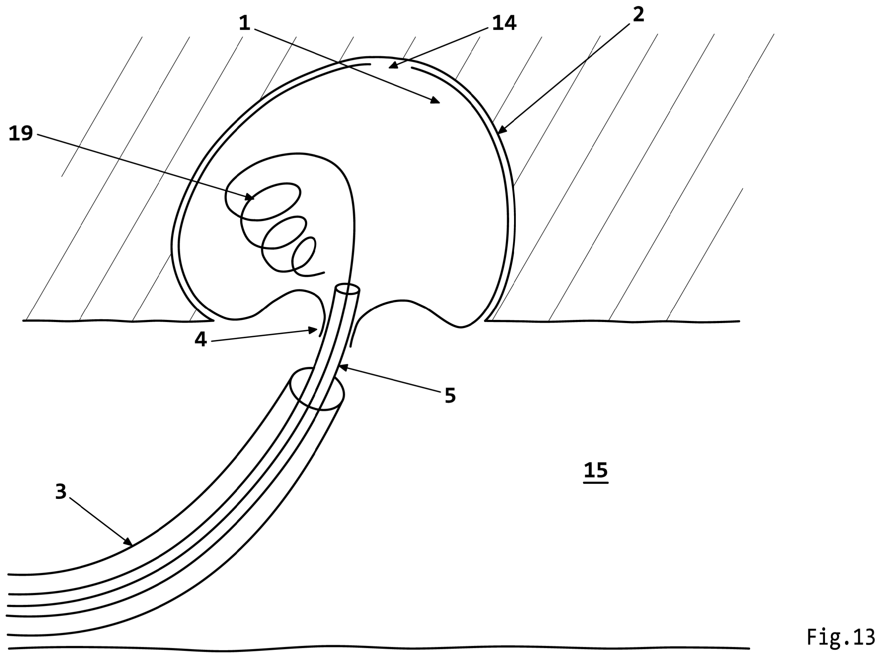

In Figur 13 wird gezeigt, wie durch die Einführhilfe 5 zusätzliche Okklusionsmittel 19 in den Innenraum des Implantats 1 eingebracht werden. Das Implantat 1 entspricht dem Implantat aus Figur 12, ist jedoch vereinfacht dargestellt. Das Implantat 1 wurde durch Zurückziehen des Mikrokatheters 3 bereits im Aneurysma 2 expandiert, wobei sich die Streben 16 des Implantats 1 an die Innenwandung des Aneurysmas 2 anlegen. Eine Ablösung des Implantats 1 hat jedoch noch nicht stattgefunden.

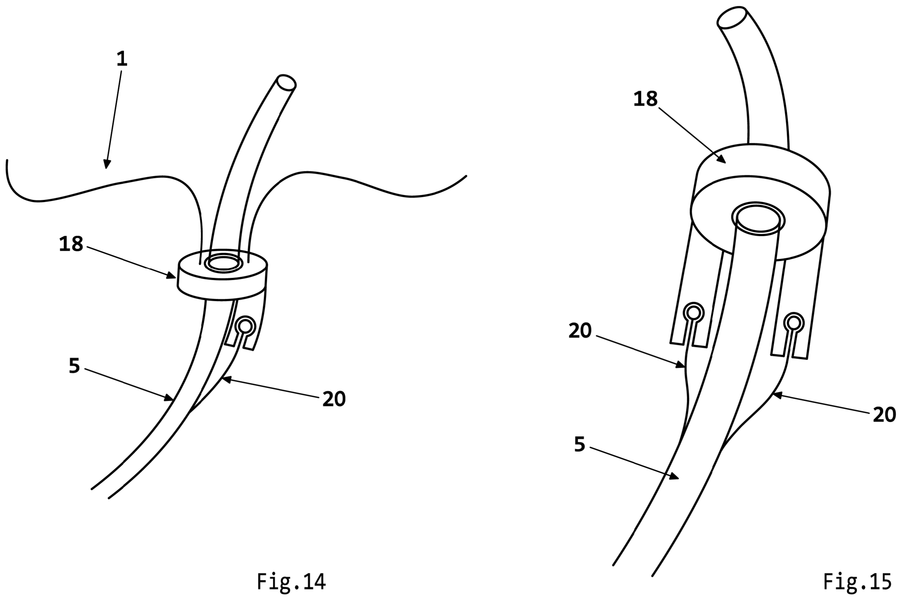

In den Figuren 14 und 15 sind verschiedene Möglichkeiten der elektrolytischen Ablösung des Implantats 1 dargestellt. In beiden Fällen ist um die schlauchförmige Einführhilfe 5 ein ringförmiges Ablöseelement 18 gelegt, welches mit dem proximalen Ende des hier nur angedeuteten Implantats 1 in Verbindung steht. Über ein (Figur 14) oder zwei (Figur 15) elektrisch leitende Ablösedrähte 20 kann ein elektrischer Strom am Ablöseelement 18 angelegt werden, der für eine elektrolytische Korrosion sorgt und somit eine Ablösung und endgültige Freisetzung des Implantats 1 bewirkt. Die Ablösedrähte 20 sind so isoliert, dass der elektrische Strom zielgerichtet an der Ablösestelle angelegt wird. Alternativ kann der Ablösedraht 20 auch durch das Lumen der Einführhilfe 5 verlaufen oder das Metallgeflecht einer katheterartigen Einführhilfe dient als entsprechender Leiter.

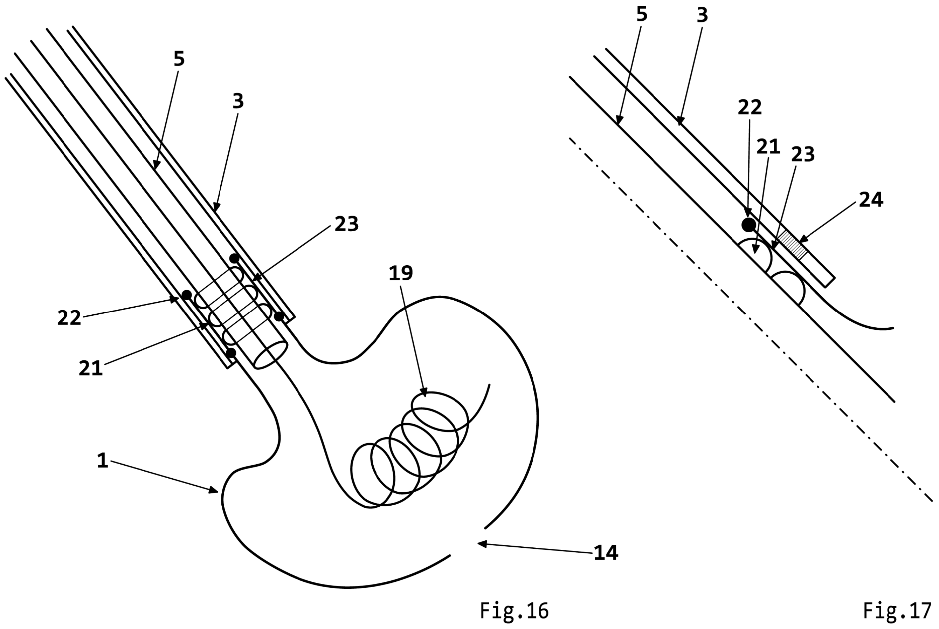

In den Figuren 16 und 17 ist eine lösbare Verbindung des Implantats 1 mit der Einführhilfe 5 dargestellt, die auf Reibschluss basiert. Um die innen hohle Einführhilfe 5 herum sind radiale Vorsprünge 21 in Form elastischer Pads angeordnet. Das proximale Ende 23 des Implantats 1, das hier aus einzelnen in proximaler Richtung vorstehenden Streben des Implantats 1 besteht, verfügt über kugelförmige Verdickungen 22 und ist zwischen Mikrokatheter 3 und Einführhilfe 5 eingeklemmt. Das Zusammenwirken von Mikrokatheter 3, Einführhilfe 5, proximalem Ende 23 des Implantats 1 sowie insbesondere der radialen Vorsprünge 21 und der Verdickungen 22 sorgt für eine reibschlüssige Verbindung und verhindert eine vorzeitige Ablösung des Implantats 1.

In Figur 17 ist das Prinzip im Detail dargestellt. Sobald der Mikrokatheter 3 in Richtung proximal (hier oben links) zurückgezogen wird, kann das proximale Ende 23 des Implantats 1 radial expandieren und das Implantat 1 wird abgelöst. Damit der behandelnde Arzt den Ablösevorgang kontrollieren kann, sind zusätzlich röntgendichte Markierungen 24 am Mikrokatheter 3 vorgesehen.

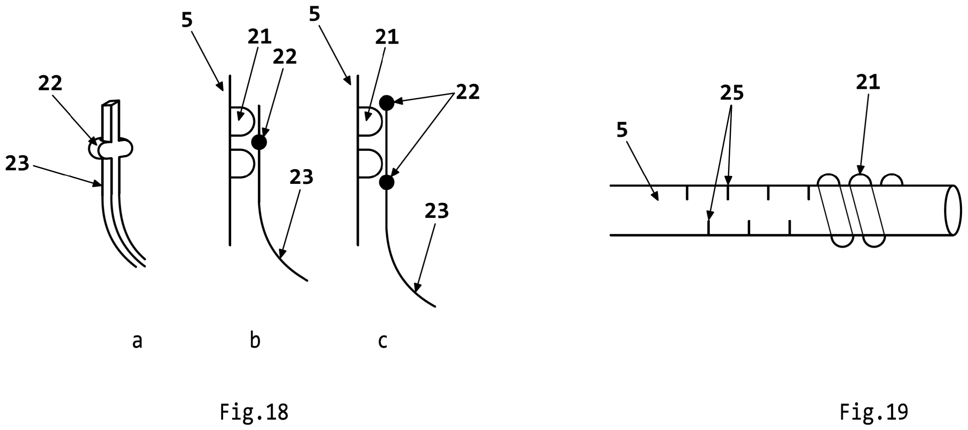

In Figur 18 a, b, c sind weitere auf Reibschluss basierende mechanisch lösbare Ablösestellen dargestellt, wobei durch entsprechende Verdickungen 22 am proximalen Ende 23 des Implantats 1 eine Kombination aus Reibschluss und Formschluss herbeigeführt wird. Die Verdickungen 22 des proximalen Endes 23 des Implantats 1 sind entweder zwischen den radialen Vorsprüngen 21 der Einführhilfe 5 angeordnet oder umgekehrt. Darum herum gelegt ist jeweils ein hier nicht dargestellter Mikrokatheter 3, der das proximale Ende 23 davon abhält, radial zu expandieren. Entsprechend kann eine Ablösung des Implantats 1 erst erfolgen, wenn der Mikrokatheter 3 nach proximal zurückgezogen wird.

In der Figur 19 ist eine schlauchförmige Einführhilfe 5 dargestellt, auf der radiale Vorsprünge 21 zur Herbeiführung des Reibschlusses angebracht sind. Zusätzlich verfügt die Einführhilfe 5 über diverse Schlitze 25, die die Flexibilität erhöhen. Dies vereinfacht den Vorschub des Implantats 1 durch den Mikrokatheter 3, insbesondere in engen Blutgefäßen.

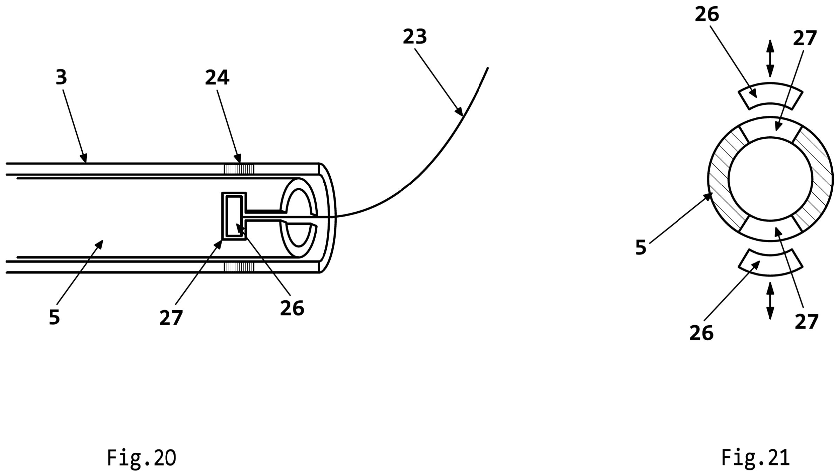

In Figur 20 ist eine formschlüssige Festlegung des proximalen Endes 23 des Implantats 1 gezeigt. Am proximalen Ende 23 sind formschlüssige Ablöseelemente 26 vorgesehen, die in entsprechende Ausnehmungen 27 in der Einführhilfe 5 eingreifen. Wenn der Mikrokatheter 3 in proximaler Richtung zurückgezogen wird, können die formschlüssigen Ablöseelemente 26 sich radial aufweiten und die Ausnehmungen 27 verlassen, wodurch eine Ablösung des Implantats 1 erfolgt. Um den Rückzug des Mikrokatheters 3 zu visualisieren, verfügt dieser zusätzlich über röntgendichte Markierungen 24.

In Figur 21 ist ein weiteres Beispiel für formschlüssige Ablöseelemente 26 gezeigt, bei dem die Ablöseelemente 26 kreissegmentförmig ausgestaltet und auf entsprechende Ausnehmungen 27 in der Einführhilfe 5 abgestimmt sind. Sobald der Mikrokatheter 3 zurückgezogen wird, können die Ablöseelemente 26 aus den Ausnehmungen 27 austreten, wie durch die Pfeile angedeutet wird.

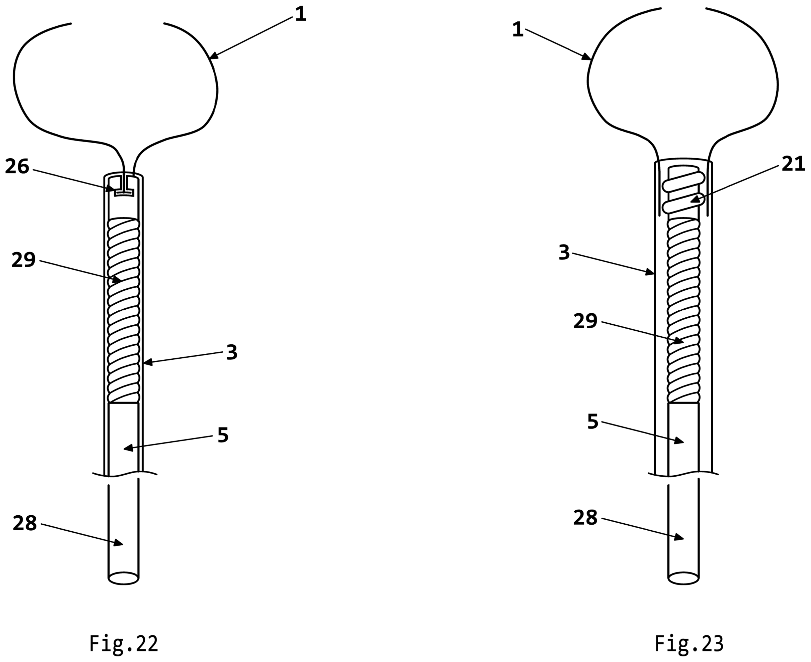

In den Figuren 22 und 23 wird gezeigt, dass die Einführhilfe 5 zweiteilig ausgebildet sein kann. Grundsätzlich entsprechen sich die Figuren 22 und 23, allerdings ist in Figur 22 eine formschlüssige Ablösung und in Figur 23 eine reibschlüssige Ablösung wie oben beschrieben dargestellt. Die Einführhilfe 5, die über ein inneres Lumen verfügt, ist im proximalen Teil 28 steifer ausgebildet als im flexibleren distalen Teil 29. Hierzu weist der distale Teil 29 eine helikale Struktur in Form eines zweilagigen Hohlstrangs auf, der über eine hohe Flexibilität verfügt. Um auch Torsionskräfte übertragen zu können, sind die Windungen der helikalen Struktur einander entgegengesetzt.

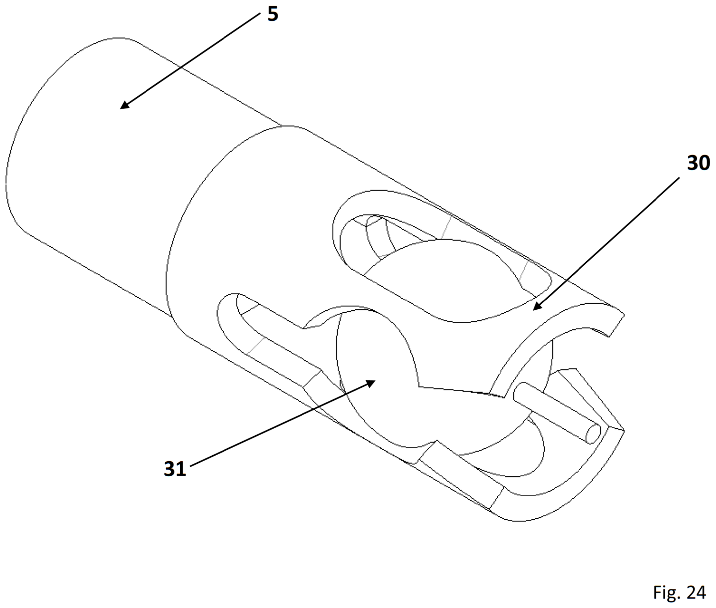

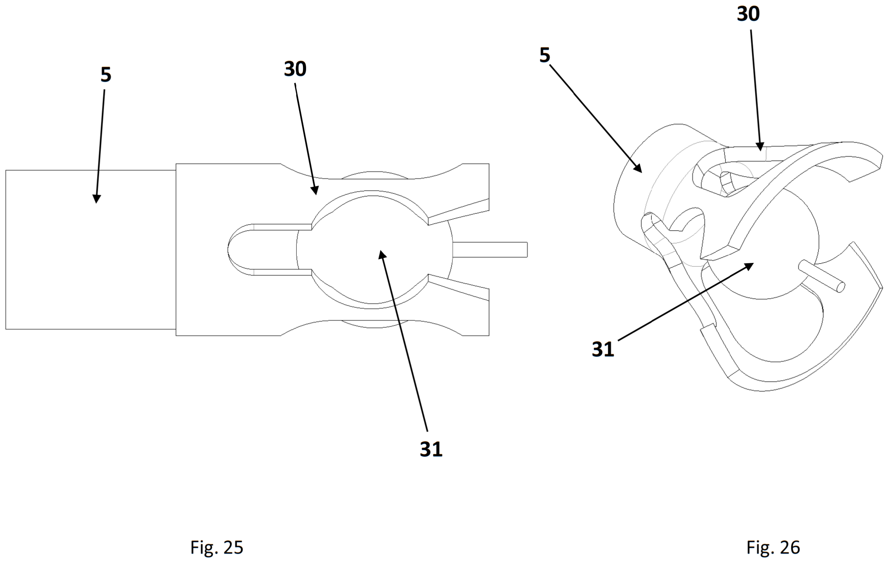

In Figur 24 ist eine weitere Variante für eine formschlüssige Festlegung des Implantats 1 an der Einführhilfe 5 dargestellt, bei der sich vom proximalen Ende des Implantats 1 ausgehend zumindest ein Ablöseelement 31 in proximaler Richtung erstreckt, das von einem an der Einführhilfe 5 angeordneten Halteelement 30 formschlüssig gehalten wird, indem es das Ablöseelement 31 umgreift. Das Ablöseelement 31 ist kugelförmig. Das Halteelement 30 ist aus einem Material mit Formgedächtniseigenschaften gefertigt. Der hier nicht dargestellte Mikrokatheter 3 ist im geschlossenen Zustand um das Halteelement 30 angeordnet und hindert dieses daran, eine aufgeweitete Sekundärstruktur einzunehmen und das Ablöseelement 31 freizugeben. Figur 25 zeigt die gleiche Situation in der Seitenansicht.

In Figur 26 ist die Situation dargestellt, nachdem der Mikrokatheter 3 in proximaler Richtung zurückgezogen wurde. Das Halteelement 30 nimmt eine ihm aufgeprägte, geöffnete Sekundärstruktur ein, sodass das Ablöseelement 31 austreten kann. Das Implantat 1 wird somit abgelöst.

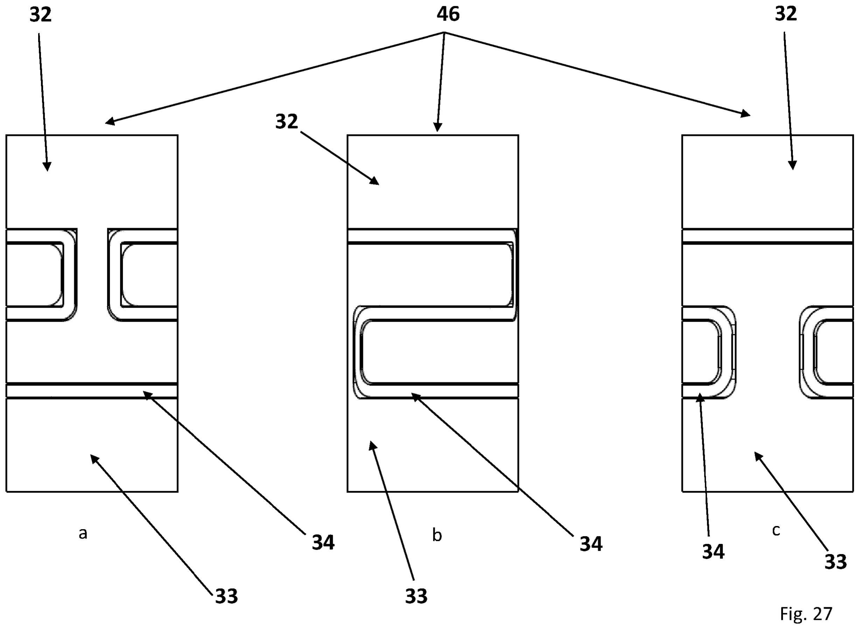

In Figur 27 ist ein Ablöseelement 46 dargestellt, das auf einer Kombination aus mechanischem Formschluss und elektrolytischer Ablösbarkeit basiert. Das Ablöseelement 46 ist von vorne (a), von der Seite (b) und von hinten (c) dargestellt. Das Ablöseelement 46 setzt sich aus 3 Komponenten zusammen, dem Einführhilfenendstück 32, das am distalen Ende der hier nicht dargestellten Einführhilfe angeordnet ist, dem Implantatendstück 33, das am proximalen Ende des hier nicht dargestellten Implantats angeordnet ist, sowie dem Isolatorelement 34, das Einführhilfenendstück 32 und Implantatendstück 33 elektrisch voneinander trennt, auch wenn diese ineinandergreifen. Einführhilfenendstück 32 und Implantatendstück 33 überlappen sich dabei, d. h. der distale Bereich des Einführhilfenendstücks 32 liegt distal des proximalen Bereichs des Implantatendstücks 33.

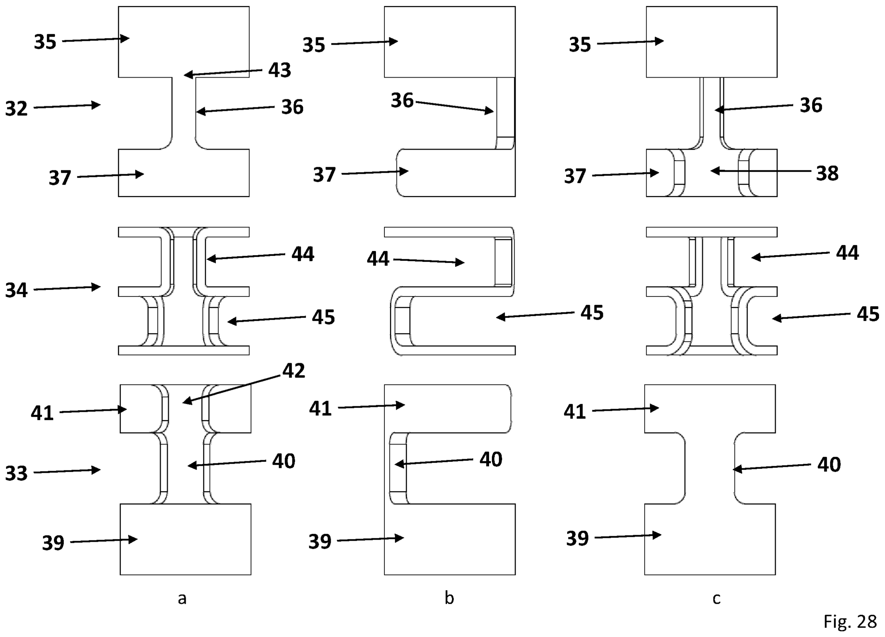

In Figur 28 sind die einzelnen Bestandteile des Ablöseelements 46 von vorne (a), von der Seite (b) und von hinten (c) dargestellt. Das Einführhilfenendstück 32 weist ein erstes kurzes Rohrelement 35 auf, welches radial geschlossen ist. Dieses ist über einen Verbindungssteg 36 mit einem zweiten kurzen Rohrelement 37 verbunden, welches radial eine Unterbrechung 38 aufweist.

Das Implantatendstück 33 ist praktisch identisch aufgebaut, d. h. es weist ein radial geschlossenes erstes kurzes Rohrelement 39, ein radial mit einer Unterbrechung 42 versehenes zweites kurzes Rohrelement 41 und einen dazwischen angeordneten Verbindungssteg 40 auf. Allerdings ist die Lage von Einführhilfenendstück 32 und Implantatendstück 33 invertiert, d. h. die mit Unterbrechungen 38, 42 versehenen zweiten Rohrelemente 37 und 41 weisen aufeinander zu, wobei die Unterbrechungen 38, 42 einen Versatz um 180° aufweisen.

Das Isolatorelement 34 verfügt über einen proximalen Abschnitt 44 und einen distalen Abschnitt 45. Das zweite Rohrelement 37 des Einführhilfenendstücks 32 greift in den distalen Abschnitt 45 ein und umschließt diesen, das zweite Rohrelement 41 des Implantatendstücks 33 hingegen greift in den proximalen Abschnitt 44 ein und umschließt diesen. Dabei sind die Ausnehmungen im proximalen und distalen Abschnitt 44, 45 auf die zweiten Rohrelemente 37, 41 abgestimmt, d. h. die Ausnehmungen werden von diesen ganz oder weitgehend ausgefüllt. Auf diese Weise wird erreicht, dass das Einführhilfenendstück 32 und das Implantatendstück 33 ineinandergreifen, dabei jedoch stets ein Bereich des Isolatorelements 34 dazwischen angeordnet ist, sodass eine an das Einführhilfenendstück 32 angelegte Spannung nicht an das Implantatendstück 33 weitergeleitet wird, sondern die beiden Endstücke 32, 33 voneinander isoliert sind.

Von Bedeutung ist darüber hinaus die elektrolytisch korrodierbar ausgebildete Ablösestelle 43, die sich bei Anlegen eines Stroms auflöst, sodass eine Trennung an dieser Stelle erfolgt. Das Implantat wird somit zusammen mit den noch anhaftenden Teilen des Ablöseelements 46 freigegeben, während das proximal gelegene erste Rohrelement 35 des Einführhilfenendstücks 32 an der Einführhilfe verbleibt und mit in Richtung proximal zurückgezogen wird. Bei der Ablösestelle 43 handelt es sich um die schmale Verbindungsstelle zwischen erstem Rohrelement 35 und Verbindungssteg 36.

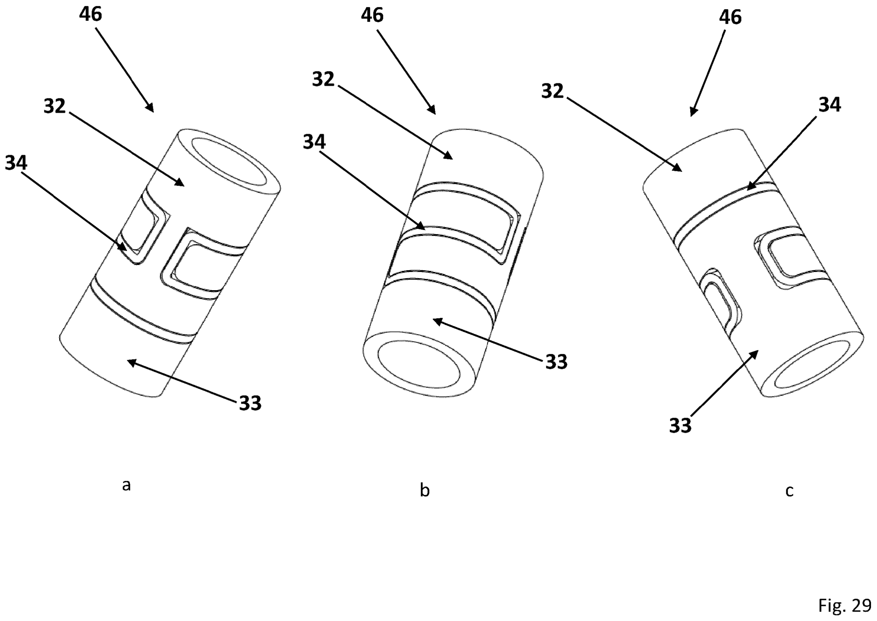

In Figur 29 ist das Ablöseelement 46 mit Einführhilfenendstück 32, Implantatendstück 33 und Isolatorelement 34 ineinandergefügt aus verschiedenen Perspektiven dargestellt. Man erkennt, wie mit Einführhilfenendstück 32 und Implantatendstück 33 formschlüssig ineinandergreifen, wobei jedoch stets Bereiche des Isolatorelements 34 einen unmittelbaren Kontakt von Einführhilfenendstück 32 und Implantatendstück 33 verhindern. Darüber hinaus erkennt man auch, dass das Ablöseelement 46 ein Lumen aufweist, das sich an das Lumen der rohr- oder schlauchförmig ausgebildeten Einführhilfe anschließt. Entsprechend ist es möglich, weitere Okklusionsmittel wie Coils oder Embolisationsmittel in das Aneurysma einzubringen.

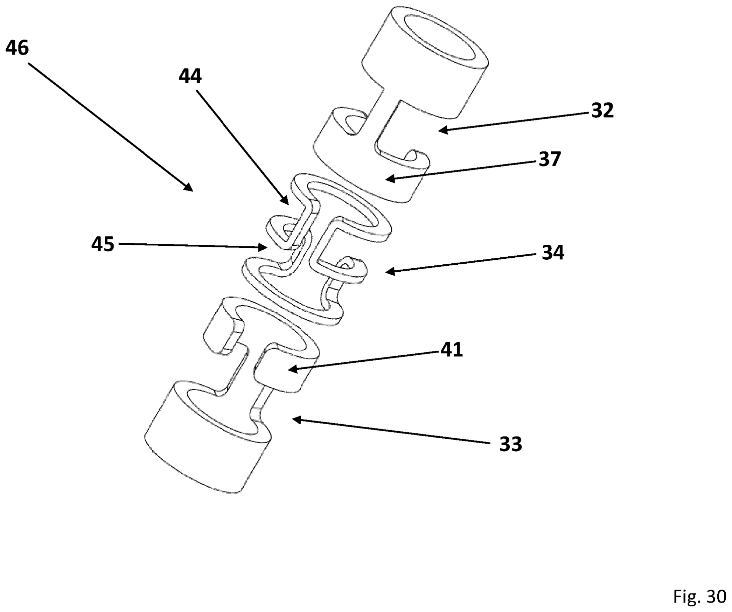

Figur 30 zeigt eine Explosionsdarstellung des Ablöseelements 46, aus der man ersieht, dass die zweiten, mit einer Unterbrechung versehenen Rohrelemente 37 und 41 von Einführhilfenendstück 32 und Implantatendstück 33 überlappend ineinandergreifen, wobei das Isolatorelement 34 dazwischen angeordnet ist. Sowohl die Unterbrechungen 38, 42 in den Endstücken 32, 33 als auch die auf die zweiten Rohrelemente 37, 41 abgestimmten Ausnehmungen im Isolatorelement 34 weisen dabei einen radialen Versatz von 180° auf.

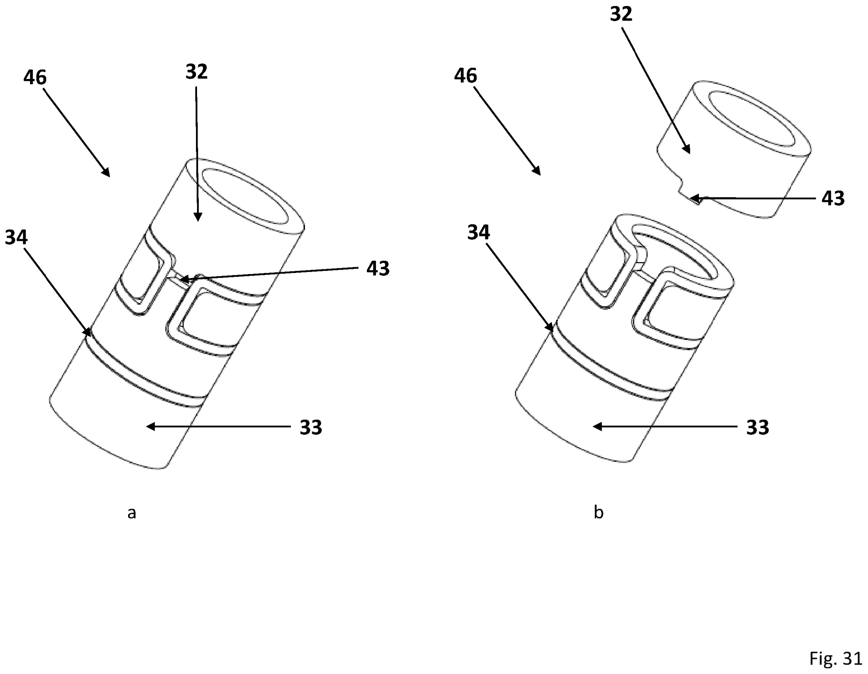

In Figur 31 schließlich wird die Ablösung des Implantats am Ablöseelement 46 dargestellt. Über die Einführhilfe und das metallische Einführhilfenendstück 32 wird ein Strom an die Ablösestelle 43 angelegt und diese als Anode geschaltet. Die korrodierbar ausgebildete Ablösestelle 43 löst sich daraufhin auf und der proximale Bereich des Einführhilfenendstücks 32 löst sich von den sonstigen Bereichen des Ablöseelements 46. Letztere werden zusammen mit dem Implantat freigesetzt, während der proximale Bereich des Einführhilfenendstücks nach proximal zurückgezogen und aus dem Blutgefäßsystem entfernt werden kann. Die am Implantat verbleibenden, metallischen Bereiche des Ablöseelements 46 können beispielsweise aus Magnesium gefertigt sein, welches sich mit der Zeit auflöst, wodurch nicht mehr benötigte Fremdkörper verschwinden. Das Ablöseelement 46 gewährleistet eine sichere formschlüssige Verbindung zwischen Einführhilfe und Implantat, die auch dann Bestand hat, wenn ein umgebender Mikrokatheter nach proximal zurückgezogen wird und das Ablöseelement 46 freigibt. Eine Freisetzung des Implantats erfolgt erst, wenn eine Spannung an der Ablösestelle 43 appliziert wird. Zudem erlaubt die formschlüssige Verbindung zwischen Einführhilfe und Implantat auch die Übertragung von Torsionsbewegungen.

**Gemäß vorteilhafter Ausführungsformen betrifft die Erfindung Objekte gemäß folgender Positionen:**
1. Implantat zur Behandlung arteriovenöser Fehlbildungen, insbesondere Aneurysmen (2), wobei das Implantat (1) in einem komprimierten Zustand durch einen Mikrokatheter (3) an einen Bestimmungsort im Blutgefäßsystem eines Patienten bringbar und dem Implantat (1) eine Sekundärstruktur aufgeprägt ist, durch die es bei Freisetzung aus dem Mikrokatheter (3) einen expandierten Zustand einnimmt, wobei das Implantat (1) über eine Ablösestelle (4) abtrennbar mit einer Einführhilfe (5) verbunden ist, wobei das Implantat (1) im expandierten Zustand einen Grundkörper (6) aufweist, der über ein proximales und ein distales Segment (7, 8) verfügt, wobei das proximale und das distale Segment (7, 8) kuppelförmig ausgebildet sind, wobei die konvexe Seite der Kuppel des proximalen Segments (7) in Richtung proximal und die konvexe Seite der Kuppel des distalen Segments (8) in Richtung distal weist, und wobei das proximale und das distale Segment (7, 8) über mehrere Verbindungsstreben (9) miteinander verbunden sind.
2. Implantat nach Position 1, wobei der Verlauf der Verbindungsstreben (9) zwischen proximalem und distalem Segment (7, 8) kurvenlinienförmig ist.
3. Implantat nach Position 1 oder 2, wobei das proximale und/oder das distale Segment (7, 8) aus miteinander zumindest teilweise verbundenen Rahmenstreben (10) aufgebaut sind.
4. Implantat nach Position 3, wobei im expandierten Zustand die Rahmenstreben (10) im proximalen und/oder distalen Segment (7, 8) eine Maschenoder Schlaufenstruktur (11) ausbilden.
5. Implantat nach Position 3 oder 4, wobei das distale Segment (8) vorzugsweise zentral einen Bereich (14) aufweist, der frei ist von Rahmenstreben (10), wobei der Bereich (14) expandierbar und komprimierbar ist.
6. Implantat nach einer der Positionen 1 bis 5, wobei das proximale Segment (7) über eine das proximale Segment (7) zumindest teilweise abdeckende Membran (12) verfügt.
7. Implantat nach Position 6, wobei das distale Segment (8) über eine das distale Segment (8) zumindest teilweise abdeckende Membran (13) verfügt.
8. Implantat nach Position 6 oder 7, wobei die Verbindungstreben (9) zumindest teilweise mit einer Membran abgedeckt sind.
9. Implantat zur Behandlung arteriovenöser Fehlbildungen, insbesondere Aneurysmen (2), wobei das Implantat (1) in einem komprimierten Zustand durch einen Mikrokatheter (3) an einen Bestimmungsort im Blutgefäßsystem eines Patienten bringbar und dem Implantat (1) eine Sekundärstruktur aufgeprägt ist, durch die es bei Freisetzung aus dem Mikrokatheter (3) einen expandierten Zustand einnimmt, wobei das Implantat (1) über eine Ablösestelle (4) abtrennbar mit einer Einführhilfe (5) verbunden ist und im expandierten Zustand einen Grundkörper (6) aufweist, der aus Streben (16) aufgebaut ist, wobei die Streben (16) zumindest teilweise untereinander an Kreuzungspunkten verbunden sind, sodass sich zwischen den Streben (16) Zwischenräume (11) ergeben, wobei sich die Streben (16) im expandierten Zustand am proximalen Ende des Grundkörpers (6) nach radial außen und im weiteren Verlauf axial in distaler Richtung und radial nach innen erstrecken, sodass sich eine Ausbauchung des Grundkörpers (6) ergibt, wobei der Grundkörper (6) am distalen Ende eine Zone aufweist, an der die Streben (16) keine Verbindung zueinander aufweisen.
10. Implantat nach Position 9, wobei der Grundkörper (6) im expandierten Zustand am distalen Ende eine Öffnung (14) aufweist.
11. Implantat nach einer der Positionen 1 bis 10, wobei der Grundkörper (6) im expandierten Zustand näherungsweise eine Kugelform, Ellipsoidform, Ovoidform, Tulpenblütenform oder die Form eines Zylinders mit konvex nach außen gewölbten Grundflächen aufweist.
12. Implantat nach einer der Positionen 1 bis 11, wobei das distale Ende der Einführhilfe (5) bei Vorliegen des Grundkörpers (6) im expandierten Zustand in dessen Innerem liegt.
13. Implantat nach einer der Positionen 1 bis 12, wobei die Einführhilfe (5) rohroder schlauchförmig ist und über ein inneres Lumen verfügt.
14. Implantat nach Position 13, wobei die rohr- oder schlauchförmige Einführhilfe (5) nach distal über die Ablösestelle (4) hinausragt.
15. Implantat nach einer der Positionen 1 bis 13, wobei außen an der Einführhilfe (5) ein Ablöseelement (18) angeordnet ist, das mit dem proximalen Ende (23) des Implantats (1) verbunden ist, wobei durch Anlegen einer elektrischen Spannung an das Ablöseelement (18) eine Freisetzung des Implantats (1) herbeiführbar ist.
16. Implantat nach Position 15, wobei das Ablöseelement (18) ringförmig um die Einführhilfe (5) angeordnet ist.
17. Implantat nach einer der Positionen 1 bis 14, wobei die Einführhilfe (5) an ihrem distalen Ende auf der Außenseite radiale Vorsprünge (21) aufweist und mit dem distalen Ende in das Implantat (1) hineinragt, sodass eine reibschlüssige Verbindung zwischen der Einführhilfe (5), dem Implantat (1) und dem Mikrokatheter (3) oder einer das distale Ende der Einführhilfe (5) umgebenden Umhüllung herbeigeführt wird und durch Bewegung des Mikrokatheters (3) oder der Umhüllung in proximaler Richtung relativ zum Implantat (1) und zur Einführhilfe eine Freisetzung des Implantats (1) erfolgt.
18. Implantat nach Position 17, wobei die radialen Vorsprünge (21) aus einem elastischen Material gefertigt sind.
19. Implantat nach Position 13 oder 14, wobei am proximalen Ende des Implantats (1) Ablöseelemente (26) angeordnet sind, die formschlüssig in hierfür vorgesehene Ausnehmungen (27) in der rohr- oder schlauchförmigen Einführhilfe (5) eingreifen, sodass durch Bewegung des Mikrokatheters (3) oder einer das distale Ende der Einführhilfe (5) umgebenden Umhüllung in proximaler Richtung relativ zum Implantat (1) und zur Einführhilfe (5) eine Ablösung des Implantats (1) erfolgt.
20. Implantat nach einer der Positionen 1 bis 14, wobei sich am proximalen Ende des Implantats (1) zumindest ein Ablöseelement (31) in proximaler Richtung erstreckt, das von einem an der Einführhilfe (5) angeordneten Halteelement (30) formschlüssig gehalten wird, wobei das Halteelement (30) aus einem Material mit Formgedächtniseigenschaften gefertigt sind und dem Halteelement (30) eine Sekundärstruktur aufgeprägt ist, bei deren Einnahme das Ablöseelement (31) freigegeben werden und eine Ablösung des Implantats (1) erfolgt, wobei das Halteelement (30) vom umgebenden Mikrokatheter (3) oder einer sonstigen das Halteelement (30) umgebenden Umhüllung an der Einnahme der Sekundärstruktur gehindert wird.
21. Implantat nach einer der Positionen 1 bis 14, wobei am distalen Ende der Einführhilfe (5) ein Einführhilfenendstück (32) angeordnet ist, das über eine elektrolytisch korrodierbare Ablösestelle (43) verfügt, wobei das Einführhilfenendstück (32) formschlüssig mit einem am proximalen Ende des Implantats (1) angeordneten Implantatendstück (33) verbunden ist und wobei ein Isolatorelement (34) in der Weise zwischen Einführhilfenendstück (32) und Implantatendstück (33) angeordnet ist, dass ein unmittelbarer Kontakt zwischen Einführhilfenendstück (32) und Implantatendstück (33) vermieden wird.
22. Implantat nach Position 21, wobei das Einführhilfenendstück (32) und das Implantatendstück (33) jeweils über ein erstes Rohrelement (35, 39) und ein zweites Rohrelement (37, 41) verfügen, wobei zwischen erstem Rohrelement (35, 39) und zweitem Rohrelement (37, 41) jeweils ein Verbindungssteg (36, 40) angeordnet ist und die zweiten Rohrelemente (37, 41) jeweils eine radiale Unterbrechung (38, 42) aufweisen, wobei das Einführhilfenendstück (32) und das Implantatendstück (33) in der Weise zusammengefügt werden, dass das zweite Rohrelement (41) des Implantatendstücks (33) proximal des zweiten Rohrelements (37) des Einführhilfenendstücks (33) angeordnet ist.

## Patentansprüche

1. Implantat zur Behandlung arteriovenöser Fehlbildungen, insbesondere Aneurysmen (2), wobei das Implantat (1) in einem komprimierten Zustand durch einen Mikrokatheter (3) an einen Bestimmungsort im Blutgefäßsystem eines Patienten bringbar und dem Implantat (1) eine Sekundärstruktur aufgeprägt ist, durch die es bei Freisetzung aus dem Mikrokatheter (3) einen expandierten Zustand einnimmt, wobei das Implantat (1) über eine Ablösestelle (4) abtrennbar mit einer Einführhilfe (5) verbunden ist und im expandierten Zustand einen Grundkörper (6) aufweist, der aus Streben (16) aufgebaut ist,
**dadurch gekennzeichnet,**
**dass** die Streben (16) zumindest teilweise untereinander an Kreuzungspunkten verbunden sind, sodass sich zwischen den Streben (16) Zwischenräume (11) ergeben, wobei sich die Streben (16) im expandierten Zustand am proximalen Ende des Grundkörpers (6) nach radial außen und im weiteren Verlauf axial in distaler Richtung und radial nach innen erstrecken, sodass sich eine Ausbauchung des Grundkörpers (6) ergibt, wobei der Grundkörper (6) am distalen Ende eine Zone aufweist, an der die Streben (16) keine Verbindung zueinander aufweisen.

2. Implantat nach Anspruch 1, **dadurch gekennzeichnet, dass** der Grundkörper (6) im expandierten Zustand am distalen Ende eine Öffnung (14) aufweist.

3. Implantat nach Anspruch 1 oder 2, **dadurch gekennzeichnet, dass** der Grundkörper (6) im expandierten Zustand näherungsweise eine Kugelform, Ellipsoidform, Ovoidform, Tulpenblütenform oder die Form eines Zylinders mit konvex nach außen gewölbten Grundflächen aufweist.

4. Implantat nach einem der Ansprüche 1 bis 3, **dadurch gekennzeichnet, dass** das distale Ende der Einführhilfe (5) bei Vorliegen des Grundkörpers (6) im expandierten Zustand in dessen Innerem liegt.

5. Implantat nach einem der Ansprüche 1 bis 4, **dadurch gekennzeichnet, dass** die Einführhilfe (5) rohr- oder schlauchförmig ist und über ein inneres Lumen verfügt.

6. Implantat nach Anspruch 5, **dadurch gekennzeichnet, dass** die rohr- oder schlauchförmige Einführhilfe (5) nach distal über die Ablösestelle (4) hinausragt.

7. Implantat nach einem der Ansprüche 1 bis 5, **dadurch gekennzeichnet, dass** außen an der Einführhilfe (5) ein Ablöseelement (18) angeordnet ist, das mit dem proximalen Ende (23) des Implantats (1) verbunden ist, wobei durch Anlegen einer elektrischen Spannung an das Ablöseelement (18) eine Freisetzung des Implantats (1) herbeiführbar ist.

8. Implantat nach Anspruch 7, **dadurch gekennzeichnet, dass** das Ablöseelement (18) ringförmig um die Einführhilfe (5) angeordnet ist.

9. Implantat nach einem der Ansprüche 1 bis 6, **dadurch gekennzeichnet, dass** die Einführhilfe (5) an ihrem distalen Ende auf der Außenseite radiale Vorsprünge (21) aufweist und mit dem distalen Ende in das Implantat (1) hineinragt, sodass eine reibschlüssige Verbindung zwischen der Einführhilfe (5), dem Implantat (1) und dem Mikrokatheter (3) oder einer das distale Ende der Einführhilfe (5) umgebenden Umhüllung herbeigeführt wird und durch Bewegung des Mikrokatheters (3) oder der Umhüllung in proximaler Richtung relativ zum Implantat (1) und zur Einführhilfe eine Freisetzung des Implantats (1) erfolgt.

10. Implantat nach Anspruch 9, **dadurch gekennzeichnet, dass** die radialen Vorsprünge (21) aus einem elastischen Material gefertigt sind.

11. Implantat nach Anspruch 5 oder 6, **dadurch gekennzeichnet, dass** am proximalen Ende des Implantats (1) Ablöseelemente (26) angeordnet sind, die formschlüssig in hierfür vorgesehene Ausnehmungen (27) in der rohr- oder schlauchförmigen Einführhilfe (5) eingreifen, sodass durch Bewegung des Mikrokatheters (3) oder einer das distale Ende der Einführhilfe (5) umgebenden Umhüllung in proximaler Richtung relativ zum Implantat (1) und zur Einführhilfe (5) eine Ablösung des Implantats (1) erfolgt.

12. Implantat nach einem der Ansprüche 1 bis 6, **dadurch gekennzeichnet, dass** sich am proximalen Ende des Implantats (1) zumindest ein Ablöseelement (31) in proximaler Richtung erstreckt, das von einem an der Einführhilfe (5) angeordneten Halteelement (30) formschlüssig gehalten wird, wobei das Halteelement (30) aus einem Material mit Formgedächtniseigenschaften gefertigt sind und dem Halteelement (30) eine Sekundärstruktur aufgeprägt ist, bei deren Einnahme das Ablöseelement (31) freigegeben werden und eine Ablösung des Implantats (1) erfolgt, wobei das Halteelement (30) vom umgebenden Mikrokatheter (3) oder einer sonstigen das Halteelement (30) umgebenden Umhüllung an der Einnahme der Sekundärstruktur gehindert wird.

13. Implantat nach einem der Ansprüche 1 bis 6, **dadurch gekennzeichnet, dass** am distalen Ende der Einführhilfe (5) ein Einführhilfenendstück (32) angeordnet ist, das über eine elektrolytisch korrodierbare Ablösestelle (43) verfügt, wobei das Einführhilfenendstück (32) formschlüssig mit einem am proximalen Ende des Implantats (1) angeordneten Implantatendstück (33) verbunden ist und wobei ein Isolatorelement (34) in der Weise zwischen Einführhilfenendstück (32) und Implantatendstück (33) angeordnet ist, dass ein unmittelbarer Kontakt zwischen Einführhilfenendstück (32) und Implantatendstück (33) vermieden wird.

14. Implantat nach Anspruch 13, **dadurch gekennzeichnet, dass** das Einführhilfenendstück (32) und das Implantatendstück (33) jeweils über ein erstes Rohrelement (35, 39) und ein zweites Rohrelement (37, 41) verfügen, wobei zwischen erstem Rohrelement (35, 39) und zweitem Rohrelement (37, 41) jeweils ein Verbindungssteg (36, 40) angeordnet ist und die zweiten Rohrelemente (37, 41) jeweils eine radiale Unterbrechung (38, 42) aufweisen, wobei das Einführhilfenendstück (32) und das Implantatendstück (33) in der Weise zusammengefügt werden, dass das zweite Rohrelement (41) des Implantatendstücks (33) proximal des zweiten Rohrelements (37) des Einführhilfenendstücks (33) angeordnet ist.
